# EUROPEAN PATENT SPECIFICATION

(11) **EP 3 227 430 B1**
(45) Date of publication and mention of the grant of the patent: **19.05.2021**
(21) Application number: 15808523.3
(22) Date of filing: 01.12.2015
(51) Int. Cl.: C12P 21/02, C12N 15/10, C12N 15/90, C12N 15/80, C12N 9/30

(54) **FUNGAL HOST STRAINS, DNA CONSTRUCTS, AND METHODS OF USE**
FADENPILZWIRTSSTÄMME, DNA-KONSTRUKTE UND VERFAHREN ZUR VERWENDUNG
SOUCHES D'HÔTES FONGIQUES, CONSTRUCTIONS D'ADN, ET MÉTHODES D'UTILISATION

(30) Priority: 01.12.2014 US 201462085834 P
(43) Date of publication of application: 11.10.2017
(73) Proprietor: Danisco US Inc., Palo Alto, California 94304 (US)
(72) Inventor: ARENTSHORST, Mark, Palo Alto, CA 94304 (US); BARENDS, Sharief, Palo Alto, CA 94304 (US); KRUITHOF, Paulien, Palo Alto, CA 94304 (US); NIKOLAEV, Igor, Palo Alto, CA 94304 (US); OUEDRAOGO, Jean Paul, Palo Alto, CA 94304 (US); RAM, Arthur F., J., Palo Alto, CA 94304 (US)
(74) Representative: Mewburn Ellis LLP
(86) International application number: PCT/US2015/063095
(87) International publication number: WO 2016/089815

(56) References cited:
- WO-A1-98/11203
- WO-A1-03/048367
- WO-A1-2011/153449
- WO-A1-2014/092960
- CN-A- 103 060 208
- DATABASE WPI Week 201364 Thomson Scientific, London, GB; AN 2013-P80009 XP002754535, & CN 103 060 208 A (CHINA JAPAN FRIENDSHIP BIOTECHNOLOGY RES) 24 April 2013 (2013-04-24)
- WATANABE S ET AL: "Mode of action of Trichoderma asperellum SKT-1, a biocontrol agent against Gibberella fujikuroi", JOURNAL OF PESTICIDE SCIENCE, vol. 32, no. 3, 27 June 2007 (2007-06-27), pages 222-228, XP055251072, ISSN: 1348-589X, DOI: 10.1584/jpestics.G06-35
- HUANG Y ET AL: "Insertion Mutagenesis of Trichoderma atroviride by Restriction Enzyme-mediated DNA integration", Journal of Shanghai Jiaotong University, vol. E-10, S1 2005, pages 161-166, XP002754546, Retrieved from the Internet: URL:http://caod.oriprobe.com/articles/3241 6556/Insertion_Mutagenesis_of_Trichoderma_ atroviride_by.htm
- ZHOU X ET AL: "Degradation of cyanide by Trichoderma mutants constructed by restriction enzyme mediated integration (REMI)", BIORESOURCE TECHNOLOGY, vol. 98, no. 15, 16 November 2006 (2006-11-16), pages 2958-2962, XP022036063, ISSN: 0960-8524, DOI: 10.1016/J.BIORTECH.2006.09.047
- WANG B ET AL: "Improved phytoremediation of oilseed rape (Brassica napus) by Trichoderma mutant constructed by restriction enzyme-mediated integration (REMI) in cadmium polluted soil", CHEMOSPHERE, vol. 74, no. 10, 23 December 2008 (2008-12-23), pages 1400-1403, XP028540471, ISSN: 0045-6535, DOI: 10.1016/J.CHEMOSPHERE.2008.11.027
- TANG J ET AL: "Improved degradation of organophosphate dichlorvos by Trichoderma atroviride transformants generated by restriction enzyme-mediated integration (REMI)", BIORESOURCE TECHNOLOGY, vol. 100, no. 1, 30 June 2008 (2008-06-30) , pages 480-483, XP025407631, ISSN: 0960-8524, DOI: 10.1016/J.BIORTECH.2008.05.022
- HE X ET AL: "Biodegradation of neonicotinoid insecticide, imidacloprid by restriction enzyme mediated integration (REMI) generated Trichoderma mutants", CHEMOSPHERE, vol. 112, 28 February 2014 (2014-02-28), pages 526-530, XP029038211, ISSN: 0045-6535, DOI: 10.1016/J.CHEMOSPHERE.2014.01.023
- SANCHEZ O ET AL: "Increased transformation frequency and tagging of developmental genes in Aspergillus nidulans by restriction enzyme-mediated integration (REMI)", MOLECULAR AND GENERAL GENETICS, vol. 258, 1 April 1998 (1998-04-01), pages 89-94, XP000986008, ISSN: 0026-8925, DOI: 10.1007/S004380050710
- DEWEI JIANG ET AL: "Molecular tools for functional genomics in filamentous fungi: Recent advances and new strategies", BIOTECHNOLOGY ADVANCES., vol. 31, no. 8, 1 December 2013 (2013-12-01), pages 1562-1574, XP055250956, GB ISSN: 0734-9750, DOI: 10.1016/j.biotechadv.2013.08.005
- BLACK M. ET AL: "Restriction enzyme-mediated integration elevates transformation frequency and enables co-transfection of Toxoplasma gondii", MOLECULAR AND BIOCHEMICAL PARASITOLOGY, vol. 74, no. 1, 1 October 1995 (1995-10-01), pages 55-63, XP009130691, ISSN: 0166-6851, DOI: 10.1016/0166-6851(95)02483-2
- KIM S. ET AL: "Genetic transformation and mutant isolation in Ganoderma lucidum by restriction enzyme-mediated integration", FEMS MICROBIOLOGY LETTERS, vol. 233, no. 2, 27 February 2004 (2004-02-27), pages 201-204, XP055538502, GB ISSN: 0378-1097, DOI: 10.1016/j.femsle.2004.02.010

## Description

### FIELD

The present disclosure relates to fungal host strains and recombinant DNA constructs for creation and use thereof. The fungal host strains are particularly stable and useful for expressing proteins of interest in a reliable or less variable fashion. The present disclosure further pertains to the use of such fungal host strain so constructed, proteins of interest and compositions comprising such proteins of interest prepared by fermentation of such improved fungal host strains.

### BACKGROUND

The use of fungal strains as production hosts for proteins of interest have long been deemed economically viable and widely used in commercial settings for some time. These proteins can then be used, optionally after being purified, in various industrial, academic or other applications. However, it is not a rare occasion when only a very small number, if any, of the transformants constructed using molecular techniques actually produce the enzyme of interest. One reason for such a small number of successful transformants may be simply the low efficiency of transformation, resulting in small number of transformants overall. Another reason is may be that a low percentage of transformants are sufficiently stable to allow production of expression products, which maybe proteins, enzymes, or other molecules.

Variability in expression of heterologous genes (e.g., non-native genes, or native genes existing in a form that is different from the native form) can occur as a consequence of factors unrelated to the genes' nucleic acid and/or amino acid sequences. For instance, non-homologous end join (NHEJ) mechanism predominates in multicellular eukaryotes, including most of fungi. Thus, expression vectors integrate into the genome at random locations, possibly resulting in positional effects on expression levels among various transformants. In addition, unstable transformants may be generated even after expression of genes of interest have been confirmed, necessitating further screening of transformants to obtain stable transformants. Variability may also occur by generation of heterokaryons as a result of transformation of a multinucleate protoplast, because the multinucleate nature of filamentous fungi hinders the targeted gene integration in these organisms. Therefore, reliable means of producing a genetically stable, transformed fungal strain to express proteins of interest, with reduced variability, provide clear advantages.

A non-limiting example of such a clear advantage lies in functionality screens of genes of interest or variants in DNA libraries expressed by fungi. Variability in expression efficacy and/or levels makes it difficult to compare the characteristics of a given variant with those of another. Therefore, a particular advantage is clearly present if genes of interest or variants can be reliably expressed by the particular host cell, and if at less variable levels such that their characteristics can be more readily assessed and compared.

Database WPI Week 201364 (Thomson Scientific, London, GB; AN) and CN103060208 disclose *Trichoderma* strain L-10 capable of efficiently expressing a beta-1, 4-glucanase coding gene glu14 sourced from *Bacillus megaterium* Ap25.

Watanabe et al (Journal of Pesticide Science, vol 32 no 3, 2007) discloses the use of *Trichoderma asperellum* SKT-1 to control the fungal disease caused by *Gibberella fujikuroi* in rice.

Huang et al (Journal of Shanghai Jiaotong University, vol. E-10, S1, 2005, pages 161-166) discloses the construction of *Trichoderma atroviride* strain T 23 through restriction enzyme-mediated integration (REMI) so as to possess hygromycin resistance

Zhou et al (BIORESOURCE TECHNOLOGY, vol. 98, no. 15, 16 November 2006, pages 2958-2962) discloses the generation of mutants with improved cyanide-degradation ability from biocontrol fungus *Trichoderma koningii* strain T30 via REMI.

Wang et al (CHEMOSPHERE, vol. 7 4, no. 10, 23 December 2008, pages 1400-1403) discloses the construction of *Trichoderma* mutants with higher Cadmium resistance through REMI.

Tang et al (BIORESOURCE TECHNOLOGY, vol. 100. no. 1, 30 June 2008, pages 480-483) discloses the use of REMI to provide *Trichoderma atroviride* strains with improved capability of degrading organophosphate pesticide dichlorvos.

Hex et al (CHEMOSPHERE, vol. 112, 28 February 2014, pages 526-530) discloses the use of REMI to construct *Trichoderma atroviride* strain T23 mutants with degrading capability of neonicotinoid insecticide, imidacloprid

WO 03/048367 discloses use in REMI of restriction enzymes with longer restriction enzyme recognition sites such as 8 or more base pairs, such as e.g. NotI, SrfI, applied to *Ashbya gossypii,* a filamentous fungus, and advantages thereof over use of restriction enzymes with shorter recognition sites.

### SUMMARY

The invention herein is as defined by the claims.

The present disclosure relates to fungal host strains and recombinant DNA constructs for creation and use thereof. The fungal host strains can be used to provide expression of genes or variants of interest in these hosts with higher reliability and/or lower variability in expression levels, as compared to other expression methods known in the art. The fungal host strains are, in a particular embodiment, useful for efficiently targeting gene integration to express genes of interest in a reliable or less variable fashion.

In a first aspect, the present disclosure provides a method of constructing a genetically stable, transformed fungal strain comprising, a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In certain embodiments, the restriction enzyme is capable of linearizing the transformed chromosomal DNA and/or generating compatible cohesive ends in the chromosomal DNA.

In some embodiments, the linearized DNA comprises one or more genes of interest. Suitably the gene of interest encodes one of hemicellulases, peroxidases, proteases, cellulases, xylanases, lipases, phospholipases, esterases, cutinases, pectinases, keratinases, reductases, oxidases, phenol oxidases, lipoxygenases, ligninases, pullulanases, tannases, pentosanases, mannanases, beta-glucanases, arabinosidases, hyaluronidases, chondroitinases, laccases, amylases, glucoamylases, or certain other useful enzymes. In some cases, the linearized DNA may comprise more than one genes of interest, and as such the transformed fungal strain expresses a mixture of products of gene encoding more than one enzymes or proteins of interest. In certain specific embodiments, the transformed fungal strain suitable expresses protein mixture. In some embodiments, the gene of interest may encode a peptide hormone, a growth factor, a clotting factor, a chemokine, a cytokine, a lymphokine, an antibody, a receptor, an adhesion molecule, a microbial antigen, or a fragment of any of such gene product.

In some embodiments, the linearized DNA further comprises at least one selective marker. The selective marker is suitably an alsl, amdS, hygR, pyr2, pyr4, pyrG, sucA, a bleomycin resistance marker, a blasticidin resistance marker, a pyrithiamine resistance marker, a chlorimuron ethyl resistance marker, a neomycin resistance marker, an adenine pathway gene, a tryptophan pathway gene, a thymidine kinase marker, or any other markers that are known to those skilled in the art or will become known to those skilled in the art as capable of being used to make the transformed fungal strain of this disclosure.

In some embodiments, the fungal strain may be an Ascomycete fungal strain.

In some embodiments, the Ascomycetes fungal strain may suitably be a filamentous fungal strain. For example, the filamentous fungal strain is a *Trichoderma,* a *Penicillium,* an *Aspergillus,* a *Humicola,* a *Chrysosporium,* a *Fusarium,* a *Neurospora,* or an *Emericella.* In certain embodiments, the filamentous fungal strain is suitably *Trichoderma reesei (T. reesei).* In alternative embodiments, the filamentous fungal strain is suitably an *Aspergillus niger.*

In a second aspect, the present disclosure provides a genetically stable, transformed fungal strain obtained using the method comprising the following steps: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In some embodiments, the genetically stable, transformed fungal strain of this aspect comprising one or more genes of interest. For instance, the gene of interest may be one encoding a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, or other useful enzymes. In some instances, the transformed fungal strain of this aspect comprises more than one genes of interest, and as such, the transformed fungal strain may produce a mixture of substances that are products of the genes of interest. In that sense, the transformed fungal strain of this aspect may be used to produce mixtures or compositions comprising products of genes of interest. In certain embodiments, the gene of interest may be one encoding a peptide hormone, a growth factor, a clotting factor, a chemokine, a cytokine, a lymphokine, an antibody, a receptor, an adhesion molecule, a microbial antigen, or a fragment of any one of such substances.

In some embodiments, the transformed fungal strain of this aspect is an Ascomycete fungal strain. In certain particular embodiments, the Ascomycetes fungal strain may suitably be a filamentous fungal strain. For instance, the filamentous fungal strain may be one from the genus *Trichoderma, Penicillium, Aspergillus, Humicola, Chrysosporium, Fusarium, Neurospora,* or *Emericella.* In certain particular embodiments, the transformed filamentous fungal strain may be a *Trichoderma reesei.* In another particular embodiment, the transformed filamentous fungal strain may suitably be an *Aspergillus niger.*

In a third aspect, the present disclosure provides a method of improving the stability of fungal transformants comprising transforming cells of a fungal strain using a mixture of linearized DNA and a restriction enzyme, wherein the restriction enzyme is capable of generating a double strand break in chromosomal DNA of said fungal strain.

In some embodiments, the restriction enzyme of the method of improving the stability of fungal transformants is capable of linearizing the transformed chromosomal DNA and/or generating compatible cohesive in the chromosomal DNA.

In some embodiments, the linearized DNA used in the method of improving the stability of fungal transformants comprises one or more genes of interest. The gene of interest is suitably one encoding a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, or the like. In certain embodiments, the linearized DNA used in the method comprises more than one genes of interest, and as such, the method can be used to produce a mixture of such enzymes, which are products encoded by the more than one genes of interest. In that sense, the method can be used to produce compositions comprising an enzyme produced by the fungal transformants with improved stability, or an enzyme or protein mixture produced by such a fungal transformant. In some further embodiments, the gene of interest may be one that encodes a peptide hormone, a growth factor, a clotting factor, a chemokine, a cytokine, a lymphokine, an antibody, a receptor, an adhesion molecule, a microbial antigen, or a fragment of such a substance.

In some embodiments, the linearized DNA of the method of this aspect further comprises at least one selective marker. For instance, the selective marker may suitably be alsl, amdS, hygR, pyr2, pyr4, pyrG, sucA, a bleomycin resistance marker, a blasticidin resistance marker, a pyrithiamine resistance marker, a chlorimuron ethyl resistance marker, a neomycin resistance marker, an adenine pathway gene, a tryptophan pathway gene, and a thymidine kinase marker.

In some embodiments, the fungal strain of this aspect is an Ascomycete fungal strain. For instance, a suitable Ascomycetes fungal strain may be a filamentous fungal strain. The filamentous fungal strain may be one of a *Trichoderma, Penicillium, Aspergillus, Humicola, Chrysosporium, Fusarium, Neurospora,* or *Emericella* strain. In some particular embodiments, the filamentous fungal strain is a *Trichoderma reesei* strain. In certain other embodiments, the filamentous strain is an *Aspergillus niger* strain.

In another aspect, the present disclosure provides a method of fermenting a transformed fungal strain in a suitable medium, which strain is obtained by the following steps: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a). In certain embodiments, the fungal strain is fermented under conditions that allow expression of a gene of interest.

In some embodiments, the gene of interest expressed by the fungal strain fermented using the method of this aspect encodes a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, or a mixture of one or more of the herein listed enzymes. In some other embodiments, the gene of interest expressed by the fungal strain can also be one that encodes a peptide hormone, a growth factor, a clotting factor, a chemokine, a cytokine, a lymphokine, an antibody, a receptor, an adhesion molecule, a microbial antigen, and one or more fragments, or mixtures of such molecules.

In certain related aspect, the disclosure also pertains to a composition that is the end-of-fermentation broth of this aspect comprising a product of the gene of interest, which is expressed by the transformed fungal strain.

In yet another aspect, the present disclosure provides a method of expressing a gene of interest comprising growing and fermenting a transformed fungal strain in a suitable medium under conditions that allow expression of said gene of interest, whereby the transformed fungal strain is obtained by the following steps: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In some embodiments, the method of this aspect further comprises assaying for the level of expression of said gene of interest. The gene of interest may suitably encode a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylases, a glucoamylase, and a mixture of one or more of these enzymes. Alternatively, the gene of interest suitably may encode a peptide hormone, a growth factor, a clotting factor, a chemokine, a cytokine, a lymphokine, an antibody, a receptor, an adhesion molecule, a microbial antigen, and fragments or mixtures of two or more of these materials.

In another aspect, the present disclosure provides a protein of interest encoded by a gene of interest, produced by a method of expressing the gene of interest in a transformed fungal cell, wherein the fungal cell is obtained by following the steps of: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In a further aspect, the present disclosure provides a proteinaceous composition comprising a protein of interest encoded by a gene of interest, whereby the gene of interest is expressed by a transformed fungal cell, which is obtained by following the steps of: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In yet a further aspect, the present disclosure provides a method of using the proteinaceous composition as produced by using any of the methods of the various aspects herein, and/or the transformed fungal strain of the disclosure, in biomass hydrolysis, cleaning applications, grain processing, animal nutrition, food composition, textile treatment and the like.

In yet another aspect, the present disclosure provides a fungal expression system, comprising: a) a fungal host cell containing at least one restriction enzyme site in chromosomal DNA; and b) a nucleic acid molecule containing a sequence operable to express a gene of interest, a selective marker and sequences with substantial homology to sequences that flank said chromosomal restriction enzyme site; wherein said sequences with substantial homology cause a homologous recombination event that results in the expression of the gene of interest.

In certain embodiments, the fungal expression system further comprises an expression cassette, which expresses a restriction enzyme that is capable of generating suitable restriction enzyme sites in the chromosomal DNA of the fungal host cell.

The gene of interest may be one encoding a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, and a mixture of two or more of these enzymes. Alternatively, the gene of interest may encode a peptide hormone, a growth factor, a clotting factor, a chemokine, a cytokine, a lymphokine, an antibody, a receptor, an adhesion molecule, a microbial antigen, a fragment or a mixture of two or more of these molecules.

In some embodiments, the selective marker may suitably be als1 amdS, hygR, pyr2, pyr4, pyrG, sucA, a bleomycin resistance marker, a blasticidin resistance marker, a pyrithiamine resistance marker, a chlorimuron ethyl resistance marker, a neomycin resistance marker, an adenine pathway gene, a tryptophan pathway gene, or a thymidine kinase marker.

In some embodiments, the fungal host strain is an Ascomycete fungal host strain. For example, the Ascomycetes fungal strain is suitably a filamentous fungal strain. In some embodiments, the fungal host strain is a *Trichoderma, Penicillium, Aspergillus, Humicola, Chrysosporium, Fusarium, Neurospora,* or *Emericella* host strain. In a particular example, the filamentous fungal host strain is a *Trichoderma reesei* host strain. In another particular example, the filamentous host strain is an *Aspergillus niger* host strain.

In a related aspect, the present disclosure provides a method of employing the fungal expression system as described herein, which fungal expression system comprises a) a fungal host cell containing at least one restriction enzyme site in chromosomal DNA; and b) a nucleic acid molecule containing a sequence operable to express a gene of interest, a selective marker and sequences with substantial homology to sequences that flank said chromosomal restriction enzyme site. The sequences with substantial homology cause a homologous recombination event, which results in the expression of the gene of interest. The nucleic acid molecule of b) is transformed into the fungal host cell in the presence of a restriction enzyme capable of generating the chromosomal restriction enzyme site.

In some embodiments, the restriction enzyme is generated by an expression cassette also situated in the fungal host cell.

In a further related aspect, the present disclosure provides a transformed or derivative fungal strain obtained by the method of employing the fungal expression system as described herein. Moreover, the disclosure also provides a method of fermenting the transformed or derivative fungal strain obtained by following the steps of: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In yet a further aspect, the present disclosure provides a method of expressing a gene of interest comprising growing and fermenting the transformed or derivative fungal strain in a suitable medium under conditions that allow expression of said gene of interest. Relatedly the disclosure also provides a protein of interest encoded by the gene of interest. Moreover, the disclosure provides a proteinaceous composition comprising the protein of interest. The disclosure also provides a method of using such proteinaceous composition in biomass hydrolysis, cleaning applications, grain processing, animal nutrition, food composition, textile treatment, or the like.

In another aspect, the present disclosure provides a method of targeted gene integration, comprising:
a) obtaining a fungal host cell comprising at least one restriction enzyme site in chromosomal DNA; and
b) obtaining a nucleic acid molecule containing a sequence operable to express one or more genes of interest, at least one selective marker and sequences with substantial homology to sequences that flank said chromosomal restriction enzyme site, wherein said homologous sequences cause a homologous recombination event that results in the expression of said gene of interest;
and transforming the nucleic acid molecule of b) into the fungal host cell of a) in the presence of a restriction enzyme capable of generating the restriction enzyme site.

In some embodiments, the fungal host cell further comprises an expression cassette, which is used to produce the restriction enzyme in the fungal host cell.

The gene of interest suitably encodes a hemicellulase, peroxidase, protease, cellulase, xylanase, lipase, phospholipase, esterase, cutinase, pectinase, keratinase, reductase, oxidase, phenol oxidase, lipoxygenase, ligninase, pullulanase, tannase, pentosanase, mannanase, beta-glucanase, arabinosidase, hyaluronidase, chondroitinase, laccase, amylase, glucoamylase, or a mixture of two of more of these enzymes. Alternatively, the gene of interest encodes a peptide hormone, a growth factor, a clotting factor, a chemokine, a cytokine, a lymphokine, an antibody, a receptor, an adhesion molecule, a microbial antigen, a fragment of these molecules or a mixture of one of more of these molecules.

The selective marker may suitably be alsl, amdS, hygR, pyr2, pyr4, pyrG, sucA, a bleomycin resistance marker, a blasticidin resistance marker, a pyrithiamine resistance marker, a chlorimuron ethyl resistance marker, a neomycin resistance marker, an adenine pathway gene, a tryptophan pathway gene, or a thymidine kinase marker.

In some embodiments, the fungal host strain is suitably an Ascomycete fungal strain. For example, the Ascomycetes fungal strain may be a filamentous fungal strain. In some embodiments, the filamentous fungal strain is a *Trichoderma, Penicillium, Aspergillus, Humicola, Chrysosporium, Fusarium, Neurospora,* or *Emericella* fungal strain. In certain specific embodiments, the filamentous fungal strain is suitably a *Trichoderma reesei* strain. In certain other specific embodiments, the filamentous fungal strain is an *Aspergillus niger* strain.

In one aspect, the present disclosure provides a method of constructing a genetically stable, transformed *Trichoderma* strain, said method comprising: a) transforming *Trichoderma* cells using a mixture of linearized (also called "linear") or circular DNA and a restriction enzyme (also called "with treatment" of the restriction enzyme), wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with treatment of the restriction enzyme is higher than percentage of stable transformants obtainable without treatment of the restriction enzyme.

In another aspect, the present disclosure provides a method of constructing a genetically stable, transformed *Trichoderma* strain, said method comprising: a) transforming *Trichoderma* cells with linearized or circular DNA, wherein the *Trichoderma* cells are induced to produce a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with induction of the restriction enzyme is higher than percentage of stable transformants obtainable without induction of the restriction enzyme.

In another aspect, the present disclosure provides a method of improving the stability of *Trichoderma* transformants comprising transforming *Trichoderma* cells using a mixture of linearized or circular DNA and a restriction enzyme, wherein the restriction enzyme is capable of generating a double strand break (DSB) in the chromosomal DNA of the *Trichoderma* cells, and wherein percentage of resulted stable transformants with treatment of the restriction enzyme is higher than percentage of stable transformants obtainable without treatment of the restriction enzyme.

In yet another aspect, the present disclosure provides a method of improving the stability of *Trichoderma* transformants comprising transforming *Trichoderma* cells with a linearized or circular DNA, wherein the *Trichoderma* cells are induced to produce a restriction enzyme that is capable of generating a double strand break (DSB) in the chromosomal DNA of the *Trichoderma* cells, and wherein percentage of resulted stable transformants with induction of the restriction enzyme is higher than percentage of stable transformants obtainable without induction of the restriction enzyme.

### BRIEF DESCRIPTION OF THE DRAWINGS

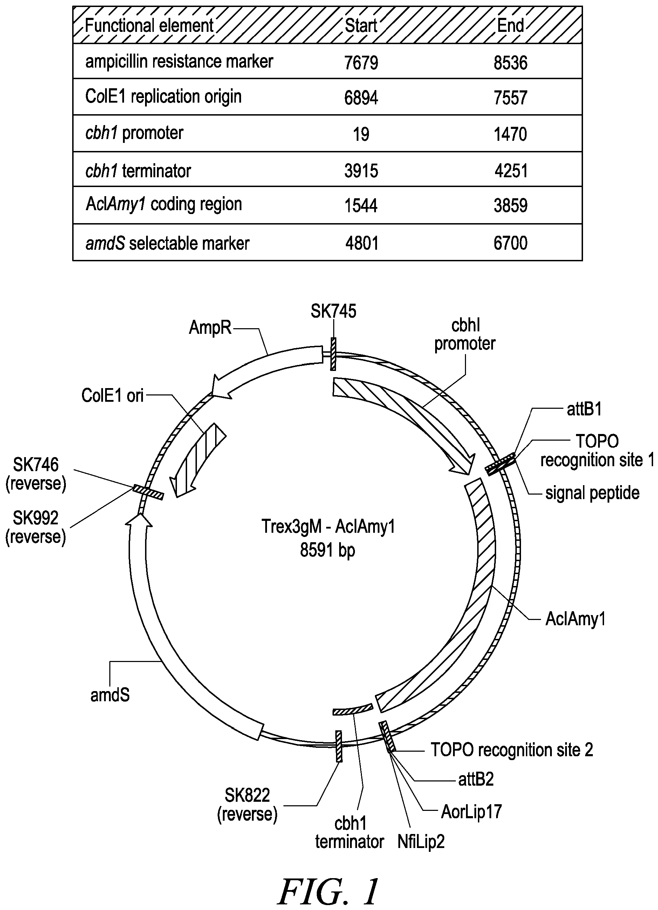

**FIG. 1****.** Vector NTI map of the AclAmy1 expression plasmid pTrex3gM-AclAmy1. This vector supported AclAmy1 expression from the genomic AclAmy1 gene driven by the *Trichoderma reesei* cellobiohydrolase 1 (*cbh1*) promoter and transcriptional terminator, as described in Example 1. It carried the *amdS* selectable marker that allows transformants to grow on acetamide as sole nitrogen source.

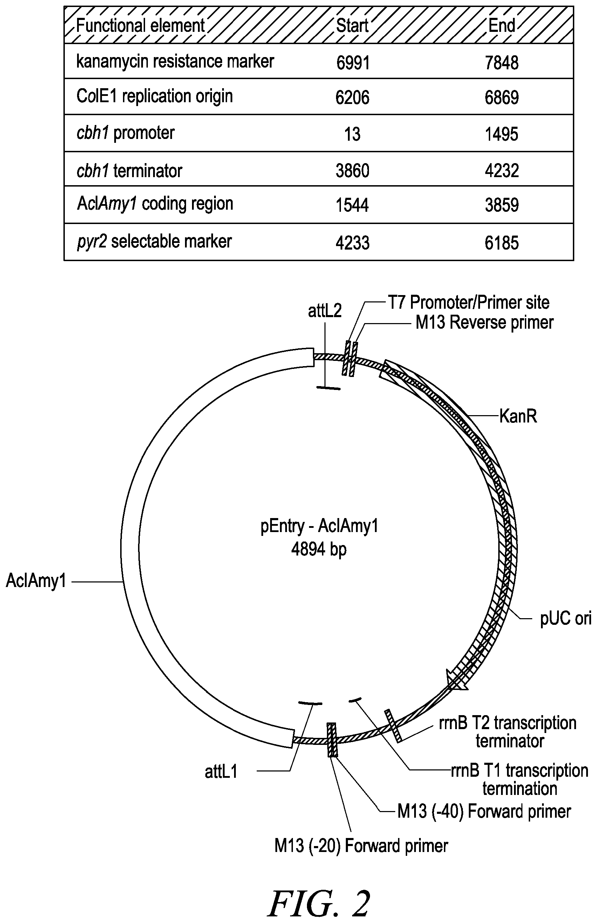

**FIG. 2****.** Vector NTI map of pENTRY-AclAmy1. The vector has been engineered as described in Example 1. It serves as a 'donor' plasmid for Gateway® LR reaction.

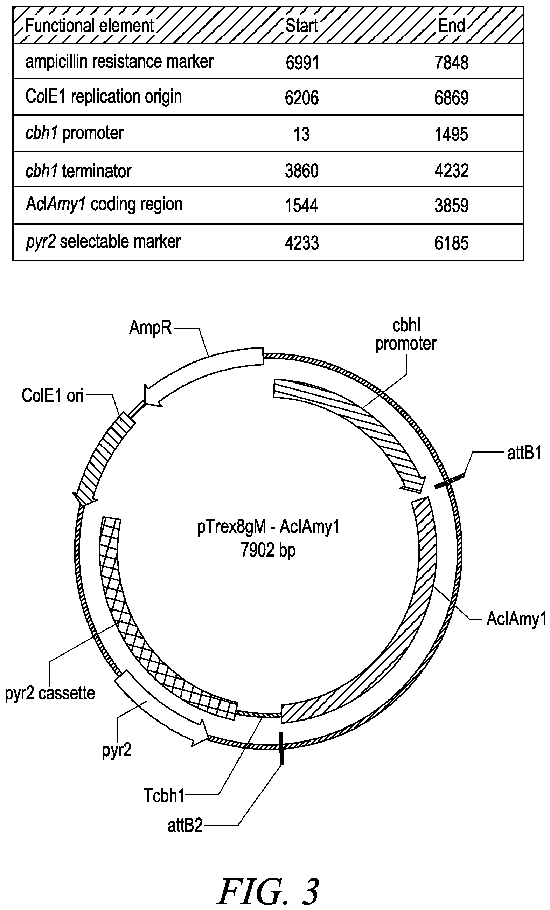

**FIG. 3****.** Vector NTI map of the AclAmy1 expression plasmid pTrex8gM-AclAmy1 for AclAmy1 expression. It supports AclAmy1 expression from the genomic AclAmy1 gene driven by the *Trichoderma reesei* cellobiohydrolase 1 (*cbh1*) promoter and transcriptional terminator. It carries the *pyr2* selectable marker that allows transformants to grow in absence of supplemented uridine.

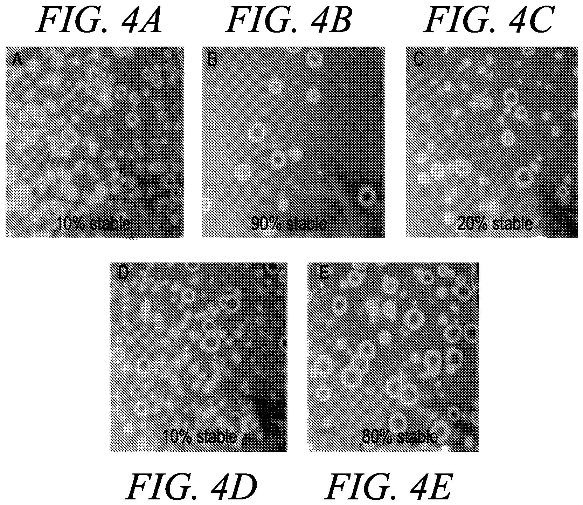

**FIGS. 4A-4E****.** Transformation of *Trichoderma* with pTrex8gMAclAmy1 (FIG. 4A), pTrex8gM-AclAmy1 in the presence of *Pac*I (FIG. 4B), the same as FIG. 4B but after column purification removing *Pac*I (FIG. 4C), PCR amplified expression construct using pTrex8gM-AclAmy1 as template (FIG. 4D), or the same as in FIG. 4D with addition of *Pac*I (FIG. 4E).

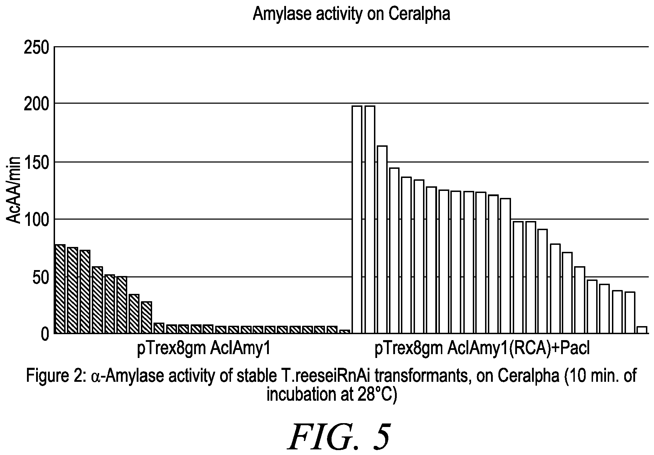

**FIG. 5****.** α-Amylase activity of stable *Trichoderma* transformants

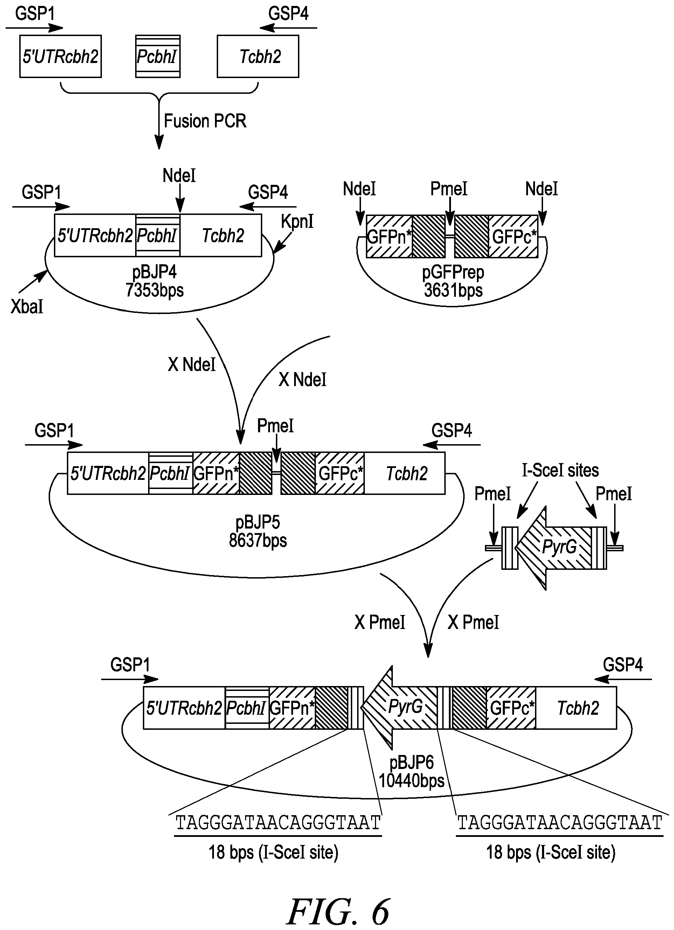

**FIG. 6****.** Construction of pBJP6 with *I-Sce*I restriction sites. ∼ 1.5 kb 5'UTR cbh2, PcbhI and Tcbh2 were PCR amplified individually, fused together by fusion PCR and cloned into *Xba*I and *Kpn*I sites of pBluescript SK(+) to generate the 7.3 kb pBJP4 plasmid. A synthetic sequence of truncated GFP repeats was inserted into the single *Nde*I restriction site of pBJP4 and a plasmid with the correct orientation of the GFP repeats was selected and named pBJP5 (8.6 Kb). The final 10 kb pBJP6 plasmid with *I-Sce*I restriction sites is obtained by cloning the PCR amplified *A. nidulans pyrG* marker surrounded by two *I-Sce*I restriction site into the single *Pme*I site of pBJP5.

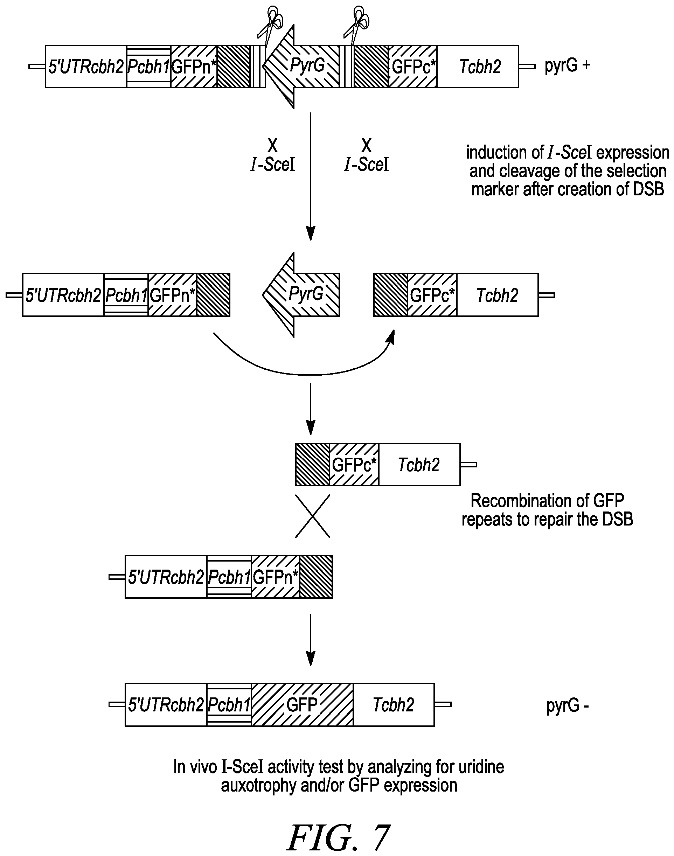

**FIG. 7****.** Rationale of the in vivo *I-Sce*I activity assay set up. The asterisk (*) represents truncated GFP sequence, and the repeated GFP sequences are shown with the more densely hatched boxes.

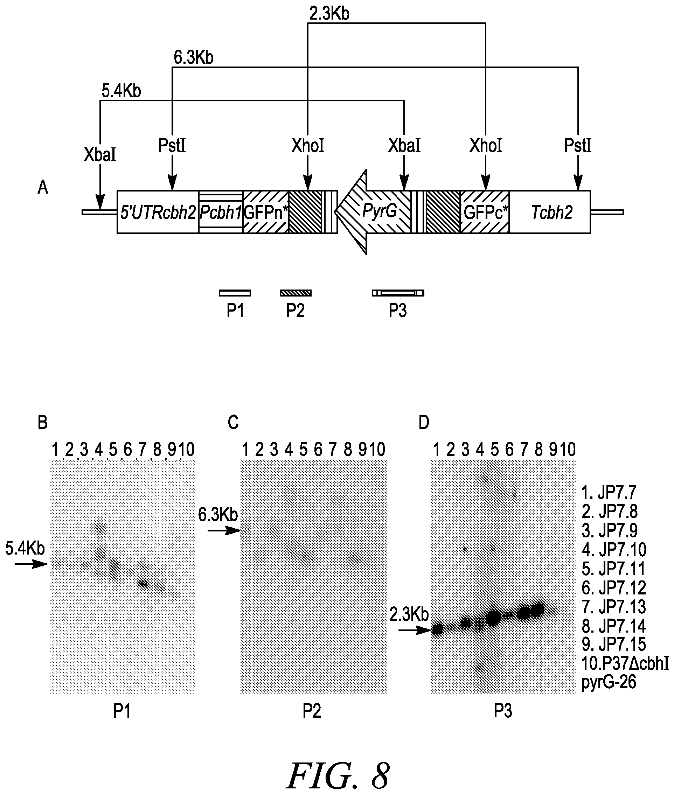

**FIG. 8****.** Introduction of the I-SceI restriction sites in the genome of *T. reesei* P37ΔcbhIpyrG-26. (A) diagram of the *I-Sce*I restriction sites cassette in the genome of *T. reesei.* The restriction enzymes used to perform the Southern blots of (B)-(D) below are indicated with vertical arrows. Three different probes (P1, P2, P3) represented by the bars at the bottom of Panel A are used to confirm the presence of the complete construct and the copy number. The size of expected restriction digestion fragments/Southern blot bands are indicated in the diagram. (B)-(D) Southern blot analysis of 9 transformants (lane 1 to 9) and the parental strain (lane 10). The strain names for samples in lanes 1-10 are indicated on the right of Panel D. (B) Genomic DNAs of the strains were digested with *Xba*I and then hybridized with probe 1. The 5.4 Kb band represents the *I-Sce*I restriction cassette when present in the genome. Number on the left indicates size of the correct band. (C) Genomic DNAs were digested with *Pst*I and hybridized with probe 2. 6.3 Kb is the expected band size of the integrated *I-Sce*I restriction sites cassette. (D) Genomic DNAs were digested with *Xho*I and hybridized with probe 3. The expecting band size with probe 3 is 2.3 Kb.

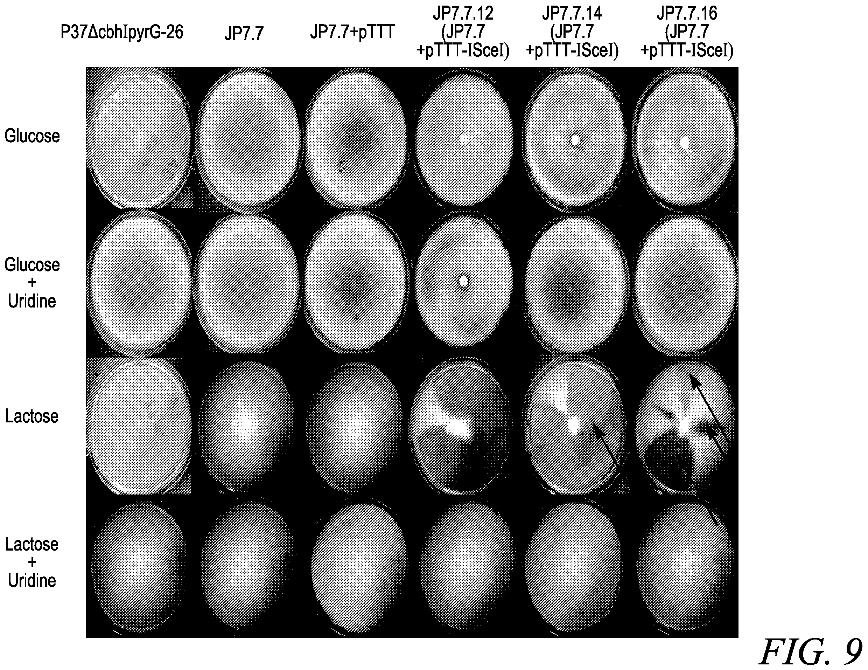

**FIG. 9****.** *In vivo I-Sce*I activity test. Induction of *I-Sce*I is predicted to create a DSB which will lead to uridine auxotrophic phenotype. Strains were point inoculated on minimal medium +/- uridine with glucose or lactose as carbon source for 4 days at 30°C. On lactose based medium expression of *I-Sce*I induces DSB which leads to loop out of the *pyrG* marker and subsequent inability of the strain to growth on the medium without uridine. Arrows indicate region with no growth.

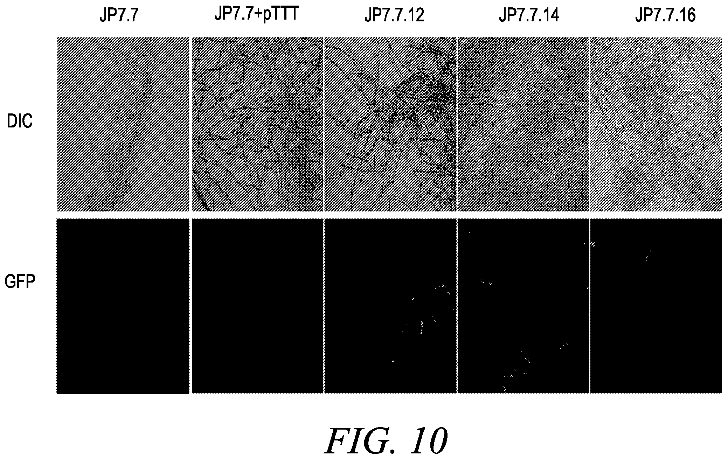

**FIG. 10****.** *In vivo I-Sce*I activity test using GFP. Induction of *I-Sce*I expression results in excision of the *pyrG* markers and restoration of GFP, indicated by the green fluorescent hyphae. Strains were grown on trMM containing lactose and uridine to induce and allow the loss of the *pyrG* marker respectively. The *Trichoderma* cells were observed by Differential Interference Contrast microscopy (DIC) for cell appearance or by fluorescence microscopy for green fluorescent protein expression (GFP).

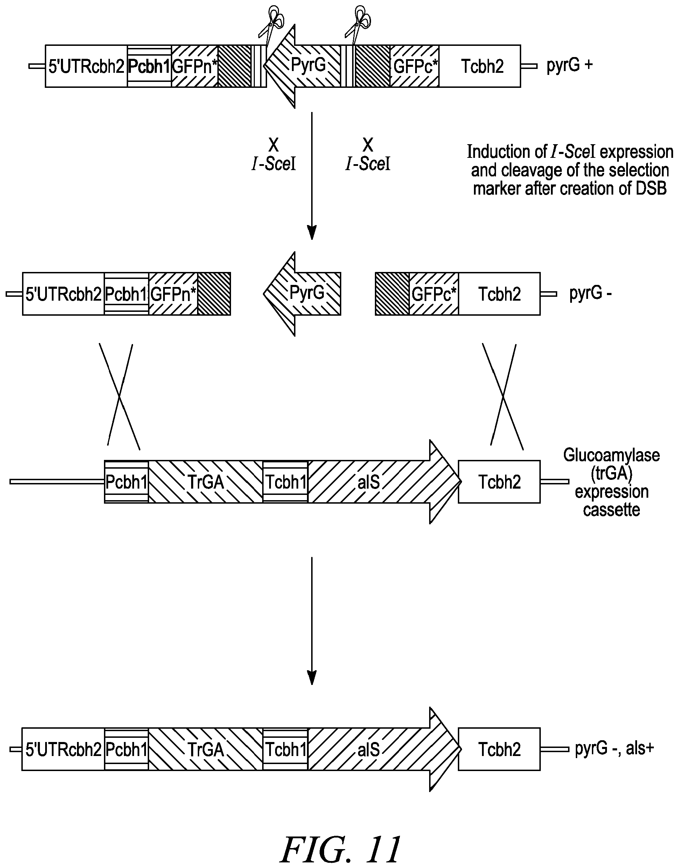

**FIG. 11****.** Rationale for targeted integration approach of the glucoamylase expression cassette mediated by *I-Sce*I*.* Targeted integration of the glucoamylase cassette at the *I-Sce*I landing site is expected to lead to alS resistance, uridine auxotrophy and glucoamylase production.

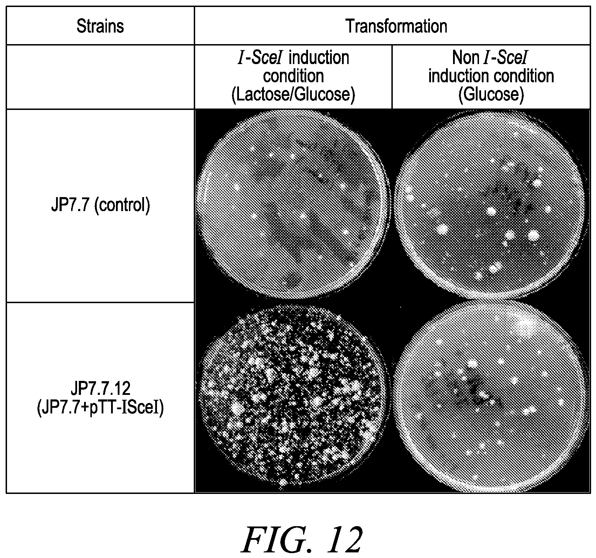

**FIG. 12****.** Increased transformation frequencies by expressing *I-Sce*I in *T. reesei.* Control strains (JP7.7) and *I-Sce*I expressing strains JP7.7.12 (JP7.7 + pTTT- *I-Sce*I) on lactose/glucose or glucose plates. Induction of *I-Sce*I via lactose increases transformation frequencies.

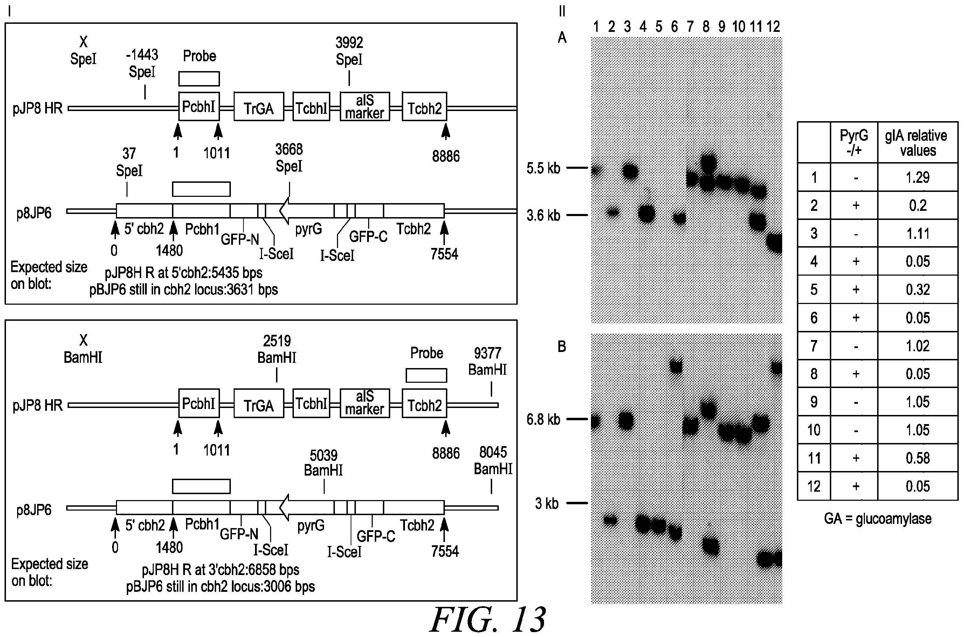

**FIG. 13****.** Southern blot analysis of selected transformant for targeted integration of the glucoamylase cassette at *I-Sce*I landing sites. (I) diagram of the Southern blot shown expected blot size when pJP8 is integration at *I-Sce*I landing sites (at the cbh2 locus) or not (pBJP6 still present at the cbh2 locus). The restriction enzymes used to perform the Southern blot are indicated by bars. Two (2) different probes (Pcbhl and Tcbh2) were used to confirm the integration at 5'flank and 3'flank respectively. The sizes of expected bands are indicated in the diagram. (II) Southern blot analysis of 12 transformants (lane 1 to 12). (II-A) Genomic DNAs of the transformants were digested with *Spe*I and then hybridized with probe Pcbhl. The 5.5 Kb band represent and homologous integration of pJP8 cassette at 5'flank and the 3.6 Kb is the expected size when the *I-Sce*I restriction sites cassette (pBJP6) is still present at the cbh2 locus. Number on the left indicates the size of the bands. (II-B) Genomic DNAs were digested with *Bam*HI and hybridized with probe Tcbh2. 6.8 Kb is the expected band size of the integrated pJP8 at 3'flank and 3Kb when not integrated. The pyrG phenotype and the relative glucoamylase activity of the selected transformants is presented in the table next to the blots picture.

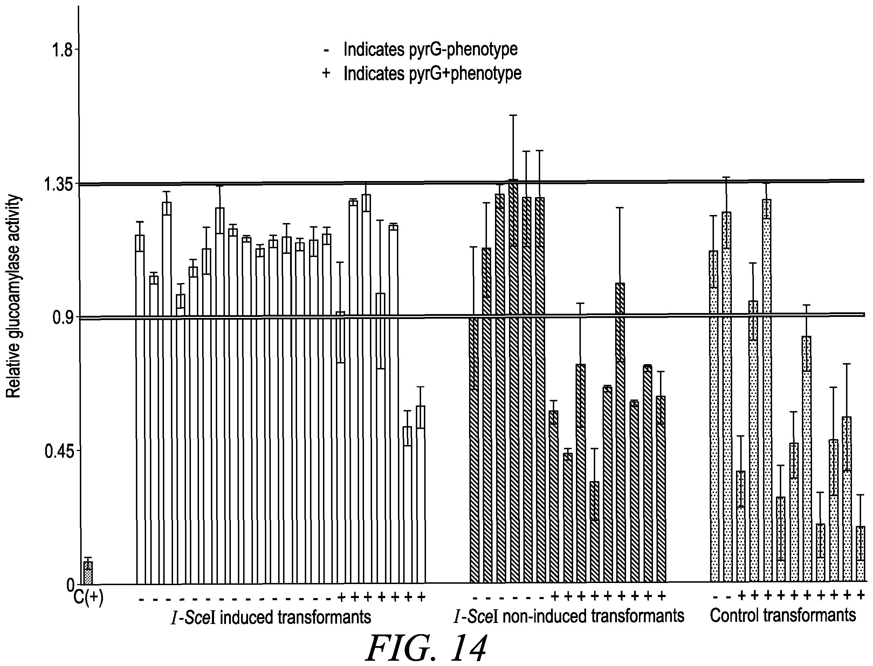

**FIG. 14****.** Comparison of glucoamylase activity between targeted (pyrG minus) and non-targeted transformants. The pyrG phenotype ("pyrG-" indicating targeted integration and "pyrG+" indicating non-targeted integration) is shown for each transformant. The glucoamylase background activity of the parental strain is represented by the solid "C" bar on the left. The open bars represent transformants expressing glucoamylase from I-SceI induced condition. The hatched bars represent transformants expressing glucoamylase from I-SceI non-induced condition, and the dotted bars represent the control transformants. The two horizontal bars (next to "1.35" and "0.9" on the y axis) delimited the relative glucoamylase activities of the targeted integrated transformants.

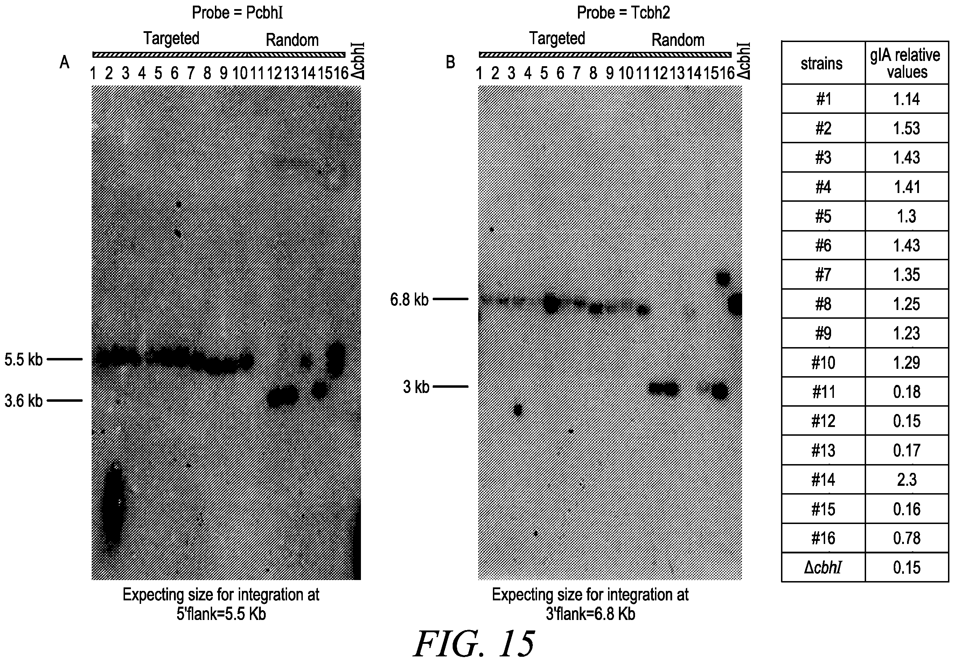

**FIG. 15****.** Southern blot analysis of targeted and non-integrated transformants of the glucoamylase cassette at *I-Sce*I landing sites. The setup of the Southern blot is identical as described in FIG. 13. (A) Genomic DNA of the transformants were digested with *Spe*I and then hybridized with probe Pcbhl. The expected correct size on blot when pJP8 is integrated at 5'flank of *I-Sce*I landing sites is 5.5 Kb. (B) Genomic DNAs were digested with *Bam*HI and hybridized with probe Tcbh2. A 6.8 Kb is the expected band size of correct integration of pJP8 at 3'flank of the *I-Sce*I landing sites. Transformants 1 to 10 are targeted integrated at the *I-Sce*I landing sites (blue bar limitation) and show less variability in glucoamylase expression. Transformants 11 to 16 are random integrated transformants (red bar limitation) and have high variability in glucoamylase expression with partial integration of the pJP8 cassette at *I-Sce*I landing site (transformants 11 and 14) or non-integration of the cassette (transformants 12, 13 and 15) or multiples copies random integration of the cassette (transformant 16). The P37ΔcbhIpyrG-26 strain is used as control in the last line of the blot and as expected there is no hybridization of this strain when using PcbhI (blot A). The expected blot size with the control using Tcbh2 as probe is 6.9 Kb (blot B).

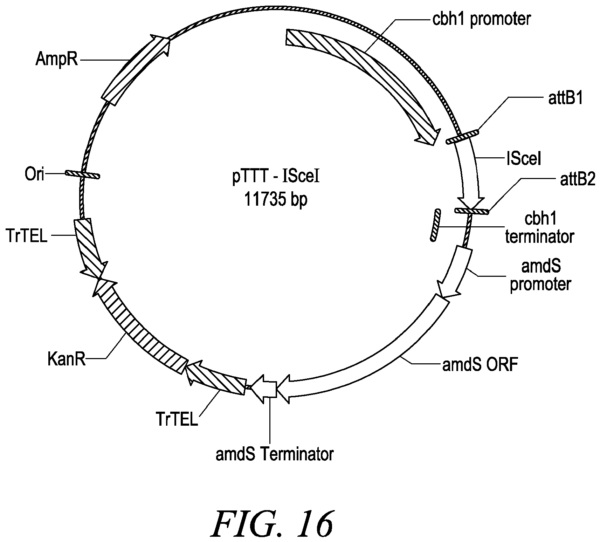

**FIG. 16****.** Plasmid map of the expression plasmid *pTTT-I-SceI.* It supports expression of *I-Sce*I from a synthetic codon optimized gene driven by the *cbhI* promoter and ended with a *cbhI* terminator. It contains the *amdS* selection marker to support growth of fungal transformants on acetamide as a sole nitrogen source. The plasmid is maintained autonomously in the fungal cell due to telomere sequence repeats on the vector.

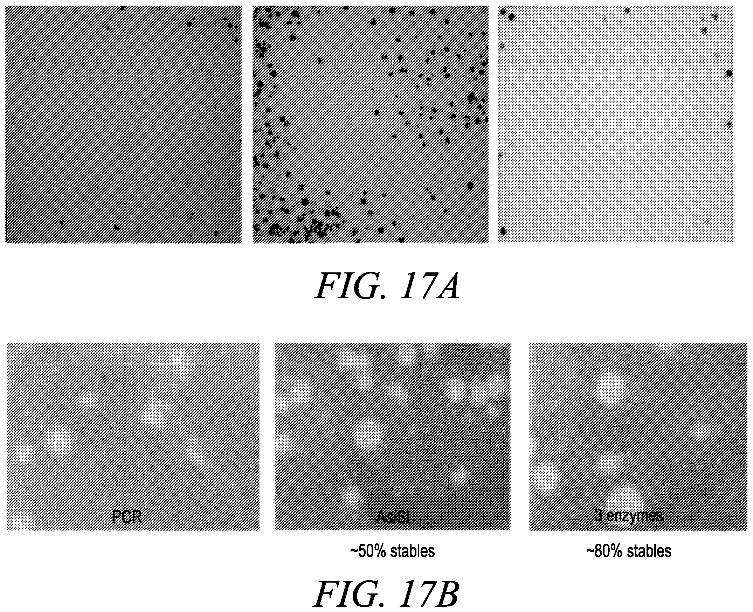

**FIGS. 17A and 17B****.** Pictures of transformation plates which show increased percentages of stable *T. reesei* transformants by treatment with multiple restriction enzymes. A *T*. *reesei* strain was transformed with a PCR fragment comprising a lytic cellulose monooxygenase (EG4)-encoding sequence without treatment with any restriction enzyme (the left panels labeled "PCR"), treated with 20 units of AsiSI (the middle panel of FIG. 17A labeled "PCR + AsiSI" and the middle panel of FIG. 17B labeled "AsiSI"), or treated with 7 units each of AsiSI, PacI and SwaI (the right panel of FIG. 17A labeled "PCR + AsiSI + PacI + Swal") or AsiSI, PacI and PmeI (the right panel of FIG. 17B labeled "3 enzymes").

### DETAILED DESCRIPTION

### I. Overview

The present disclosure relates to fungal host strains and recombinant DNA constructs for creation and use thereof. The fungal host strains are particularly stable and useful for expressing proteins of interest in a reliable or less variable fashion. The present disclosure further pertains to the use of such fungal host strain so constructed, proteins of interest and compositions comprising such proteins of interest prepared by fermentation of such improved fungal host strains.

The methods described herein express proteins of interest or variants of interest with improved reliability. The term "improved reliability" thus means that (1) at least 60% (e.g., at least 65%, at least 70%, at least 75%, at least 80%, at least 85%, at least 90%, at least 95%, or at least 99%) of the transformants are stable transformants; or (2) that at least 60% (e.g., at least 65%, at least 70%, at least 75%, at least 80%, at least 85%, at least 90%, at least 95%, or at least 99%) of the transformants express the protein or variant of interest as intended over the background expression level; or (3) that the proteins or variants of interest are expressed with expression levels varying less than 60% (e.g., less than 55%, less than 50%, less than 45%, less than 40%, less than 35%, less than 30%, less than 25%, less than 20%, less than 15%, less than 10%, less than 5%, or less than 2%). The term "expression level variation" as mentioned in (3) above or elsewhere herein, is defined or determined by dividing the difference between the highest and the lowest expression levels with a value that is the difference between the highest expression level and the background expression level, wherein all determination are made with the same construct and the same gene or variant of interest.

### II. Definitions

Prior to describing the present strains, compositions and methods, the following terms and phrases are defined. Terms not defined should be accorded their ordinary meaning as used in the art.

Where a range of values is provided, it is understood that each intervening value, to the tenth of the unit of the lower limit unless the context clearly dictates otherwise, between the upper and lower limit of that range and any other stated or intervening value in that stated range, is encompassed within the present compositions and methods. The upper and lower limits of these smaller ranges may independently be included in the smaller ranges and are also encompassed within the present compositions and methods, subject to any specifically excluded limit in the stated range. Where the stated range includes one or both of the limits, ranges excluding either or both of those included limits are also included in the present compositions and methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating un-recited number may be a number which, in the context in which it is presented, provides the substantial equivalent of the specifically recited number. For example, in connection with a numerical value, the term "about" refers to a range of - 10% to +10% of the numerical value, unless the term is otherwise specifically defined in context. In another example, the phrase a "pH value of about 6" refers to pH values of from 5.4 to 6.6, unless the pH value is specifically defined otherwise.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages, and so forth.

It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The term "recombinant," when used in reference to a subject cell, nucleic acid, polypeptides/enzymes or vector, indicates that the subject has been modified from its native state. Thus, for example, recombinant cells express genes that are not found within the native (non-recombinant) form of the cell, or express native genes at different levels or under different conditions than found in nature. Recombinant nucleic acids may differ from a native sequence by one or more nucleotides and/or are operably linked to heterologous sequences, *e.g.,* a heterologous promoter, signal sequences that allow secretion, etc., in an expression vector. Recombinant polypeptides/enzymes may differ from a native sequence by one or more amino acids and/or are fused with heterologous sequences. A vector comprising a nucleic acid encoding a protein of interest is, for example, a recombinant vector.

It is further noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising," as used herein, means including, but not limited to, the component(s) after the term "comprising." The component(s) after the term "comprising" are required or mandatory, but the composition comprising the component(s) may further include other non-mandatory or optional component(s).

It is also noted that the term "consisting of," as used herein, means including, and limited to, the component(s) after the term "consisting of." The component(s) after the term "consisting of' are therefore required or mandatory, and no other component(s) are present in the composition.

As will be apparent to those of skill in the art upon reading this disclosure, each of the individual embodiments described and illustrated herein has discrete components and features which may be readily separated from or combined with the features of any of the other several embodiments without departing from the scope of the present compositions and methods described herein. Any recited method can be carried out in the order of events recited or in any other order which is logically possible.

As used herein, the term "genetically stable stains" refers to their faster growth rate in comparison to that of the unstable strain. Also, with a number of filamentous fungal hosts, such as, for example, *Trichoderma,* the formation of circular colonies with a smooth, as opposed to a ragged outline on solid culture medium can be used as a distinguishing feature.

As used herein, the term "protein of interest" refers to a polypeptide that is desired to be expressed in a filamentous fungus, optionally at high levels and for the purpose of commercialization. Such a protein may be an enzyme, a substrate-binding protein, a surface-active protein, a structural protein, or the like.

As used herein, the term "gene of interest" refers to gene coding sequence of protein of interest.

As used herein, the term "vector" refers to a nucleic acid construct designed for transfer between different host cells. An "expression vector" refers to a vector that has the ability to incorporate and express heterologous DNA fragments in a foreign cell. Many prokaryotic and eukaryotic expression vectors are commercially available. Selection of appropriate expression vectors is within the knowledge of those having skill in the art.

Accordingly, an "expression cassette" or "expression vector" is a nucleic acid construct generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter.

As used herein, the term "plasmid" refers to a circular double-stranded (ds) DNA construct used as a cloning vector, and which forms an extra chromosomal self-replicating genetic element in many bacteria and some eukaryotes.

As used interchangeably herein, the term "selective marker" or "selectable marker" refers to a gene natively present or introduced into a cell whose expression confers to the cell the ability to grow in the presence of a corresponding selective agent, or under corresponding selective growth conditions. As used herein, the term "selectable marker-encoding nucleotide sequence" refers to a nucleotide sequence which encodes such selective marker or selectable marker.

As used herein, the term "promoter" refers to a nucleic acid sequence that functions to direct transcription of a downstream gene. The promoter will generally be appropriate to the host cell in which the target gene is being expressed. The promoter, together with other transcriptional and translational regulatory nucleic acid sequences (also termed "control sequences"), are often used to express a given gene. In general, the transcriptional and translational regulatory sequences include, but are not limited to, promoter sequences, ribosomal binding sites, transcriptional start and stop sequences, translational start and stop sequences, and enhancer or activator sequences.

As used herein, the terms "polypeptide" and "protein" are used interchangeably to refer to polymers of any length comprising amino acid residues linked by peptide bonds. The conventional one-letter or three-letter codes for amino acid residues are used herein. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

As used herein, "fungus" is any member of a large group of eukaryotic organisms that includes microorganisms such as yeasts and molds, as well as the mushrooms. These organisms are classified as a kingdom, Fungi, which are separate from plants, animals, protists, and bacteria. One major difference is that fungal cells have cell walls that contain chitin, unlike the cell walls of plants and some protists, which contain cellulose, and unlike the cell walls of bacteria.

As used herein, "Ascomycete fungal strain", refers to any organism in the Division Ascomycota in the Kingdom Fungi. Exemplary Ascomycetes fungal cells include but are not limited to filamentous fungi in the subphylum *Pezizomycotina,* such as *Trichoderma spp, Aspergillus spp,* and *Penicillium spp.*

As used herein, the "filamentous fungus" refers to all filamentous forms of the subdivision Eumycota and Oomycota. For example, filamentous fungi include, without limitation, *Acremonium, Aspergillus, Emericella, Fusarium, Humicola, Mucor, Myceliophthora, Neurospora, Scytalidium, Thielavia, Tolypocladium,* or *Trichoderma* species. In some embodiments, the filamentous fungus may be an *Aspergillus aculeatus, Aspergillus awamori, Aspergillus foetidus, Aspergillus japonicus, Aspergillus nidulans, Aspergillus niger,* or *Aspergillus oryzae.* In some embodiments, the filamentous fungus is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum.* In some embodiments, the filamentous fungus is a *Humicola insolens, Humicola lanuginosa, Mucor miehei, Myceliophthora thermophila, Neurospora crassa, Scytalidium thermophilum,* or *Thielavia terrestris.* In some embodiments, filamentous fungus is a *Trichoderma harzianum, Trichoderma koningii, Trichoderma longibrachiatum, Trichoderma reesei,* e.g., RL-P37 (Sheir- Neiss et al., Appl. Microbiol. Biotechnology, 20 (1984) pp. 46-53; Montenecourt B.S., Can., 1- 20, 1987), QM9414 (ATCC No. 26921), NRRL 15709, ATCC 13631, 56764, 56466, 56767, or *Trichoderma viride,* e.g., ATCC 32098 and 32086. In some embodiments, the filamentous fungus is a *Trichoderma reesei* RutC30, which is available from the American Type Culture Collection as *Trichoderma reesei* ATCC 56765. Related to this, in some embodiments, the disclosure provides a whole cell broth preparation of any one of the filamentous fungi described herein.

A "heterologous" nucleic acid construct or sequence has a portion of the sequence which is not native or existing in a native form to the cell in which it is expressed. Heterologous, with respect to a control sequence refers to a control sequence (i.e. promoter or enhancer) that does not function in nature to regulate the same gene the expression of which it is currently regulating. Generally, heterologous nucleic acid sequences are not endogenous to the cell or part of the genome in which they are present, and have been added to the cell, by infection, transfection, transformation, microinjection, electroporation, or the like. A "heterologous" nucleic acid construct may contain a control sequence/DNA coding sequence combination that is the same as, or different from a control sequence/DNA coding sequence combination found in the native cell.

The term "host cell", as used herein, includes any fungus, whether a unicellular organism, a cell derived from a multicellular organism and placed in tissue culture or a cell present as part of a multicellular organism, which is susceptible to transformation with a nucleic acid construct according to the disclosure. Such host cells, such as yeast and other fungal cells may be used for replicating DNA and producing polypeptides encoded by nucleotide sequences as used in the disclosure. Suitable cells are generally filamentous fungi or yeasts. Particularly preferred are cells from filamentous fungi, preferably Aspergillus, such as A. *niger* and A. *tubingensis.* Other preferred organisms include any one of *Aspergillus oryzae, A. awamori, Trichoderma reesei, T. viride* and *T. longibrachiatum.*

As used herein, "transformed" means a cell has been transformed by use of recombinant DNA techniques. Transformation typically occurs by insertion of one or more nucleotide sequences into a cell. The inserted nucleotide sequence may be a heterologous nucleotide sequence, i.e., is a sequence that is not natural to the cell that is to be transformed, such as a fusion protein.

The term "derivative" encompasses the terms "originated" "obtained," "obtainable," and "created," and generally indicates that one specified material find its origin in another specified material or has features that can be described with reference to the another specified material.

As used herein, the term "gene" means the segment of DNA involved in producing a polypeptide, that may or may not include regions preceding and following the coding region, e.g. 5' untranslated (51 UTR) or "leader" sequences and 3' UTR or "trailer" sequences, as well as intervening sequences (introns) between individual coding segments (exons). In other embodiments, the gene encodes commercially important industrial proteins or peptides, such as enzymes, e.g., *proteases, mannanases, xylanases, amylases, glucoamylases, cellulases, oxidases and lipases.* The gene of interest may be a naturally occurring gene, a mutated gene or a synthetic gene.

As used interchangeably herein, the term "restriction enzyme", "restriction endonuclease", or "site-specific endonuclease" is an enzyme that cuts DNA at or near specific recognition nucleotide sequences known as restriction sites. Restriction enzymes are commonly classified into three types, which differ in their structure and whether they cut their DNA substrate at their recognition site, or if the recognition and cleavage sites are separate from one another. Type I can cleave at random sites of about 1000 base pairs or more from the recognition sequence. Type II's cleavage sequence overlaps with its recognition sequence. Type III cleaves DNA at about 25 base pairs from the recognition sequence. Also classified under restriction enzymes are meganucleases, Zinc-finger nucleases (ZFNs), Transcription activator-like effector nucleases (TALENs), and CRISPR/Cas such as RNA-guided endonucleases (e.g., Cas9). Meganucleases are naturally occurring restriction enzymes that recognize large recognition sites of about12-40 base pairs which are very rare in genomes. Examples of meganucleases include homing meganucleases such as the LAGLIDADG family, examples of which including I-SceI, I-CreI, I-MsoI, I-AniI, I-DmiI, and PI-PfuI. Zinc-finger nucleases (ZFNs) are fusions of the non-specific DNA cleavage domain from the FokI restriction endonuclease with zinc-finger proteins. ZFN dimers induce targeted DNA double-strand breaks (DSBs) that stimulate DNA damage response pathways. The binding specificity of the designed zinc-finger domain directs the ZFN to a specific genomic site. Transcription activator-like effector (TALE) nucleases (TALENs) are fusions of the Fokl cleavage domain and DNA-binding domains derived from TALE proteins. TALEs contain multiple 33-35 amino acid repeat domains that each recognizes a single base pair. Like ZFNs, TALENs induce targeted DSBs that activate DNA damage response pathways and enable custom alterations. RNA-guided endonucleases (e.g., Cas9) are part of CRISPR/Cas systems. CRISPR, or Clustered Regulatory Interspaced Short Palindromic Repeats, are loci that contain multiple short direct repeats, and provide acquired immunity to bacteria and archaea. CRISPR systems rely on crRNA and tracrRNA for sequence-specific silencing of invading foreign DNA. Three types of CRISPR/Cas systems exist. In type II systems, Cas9 serves as an RNA-guided DNA endonuclease that cleaves DNA upon crRNA-tracrRNA target recognition.

To cut DNA, all restriction enzymes make two incisions, once through each sugar-phosphate backbone (i.e. each strand) of the DNA double helix. Over 3,000 restriction enzymes have been studied in detail, and more than 600 of these are available commercially. These enzymes are routinely used for DNA modification in laboratories, and are a vital tool in molecular cloning. For example, the following restriction enzymes recognize 8-base-pair restriction sites and are available commercially: PacI, Swal, PmeI, AscI, AsiSI, FseI, NotI, SrfI, and Sgfl. The restriction enzymes used herein include both naturally occurring restriction enzymes and synthetic, hybrid, or mutant restriction enzymes, as long as they can generate a double strand break (DSB) in the chromosomal DNA of a filamentous fungus.

As used herein, the term "restriction enzyme site", "restriction sites" or "recognition sites" are locations on a DNA molecule containing specific (from 4-8 base pairs to 12-40 base pairs in length) sequences of nucleotides, which are recognized by various restriction enzymes. These could be either palindromic sequences (because many restriction enzymes bind as homodimers), or asymmetrical (for tailor designed TALENs or ZNF nucleases) and a particular restriction enzyme may cut the sequence between two nucleotides within its recognition site, somewhere nearby, or far away such as more than 1000 bp away. For example, the common restriction enzyme *EcoR*I recognizes the palindromic sequence GAATTC and cuts between the G and the A on both the top and bottom strands, leaving an overhang of AATT on each end. An overhang is an end-portion of a DNA strand with no attached complement, also known as a sticky or cohesive end. This overhang can then be used to ligate in (see DNA ligase) a piece of DNA with a complementary overhang, also known as compatible cohesive end (another *EcoR*I-cut piece, for example). Some restriction enzymes cut DNA at a restriction site in a manner which leaves no overhang, called a blunt end.

As used herein, the term "% identity" is used interchangeably with the term "% homology" and refers to the level of nucleic acid or amino acid sequence identity between the nucleic acid sequences that encode any one of the inventive polypeptides or the inventive polypeptide's amino acid sequences, when aligned using a sequence alignment program.

### III. Fungal host strain and DNA constructs, and methods of use thereof

The present disclosure relates to fungal host strains and recombinant DNA constructs for creation and use thereof. The fungal host strains can be used to provide expression of genes or variants of interest in these hosts with higher reliability and/or lower variability in expression levels, as compared to other expression methods known in the art. The fungal host strains are, in a particular embodiment, useful for efficiently targeting gene integration to express genes of interest in a reliable or less variable fashion.

### 1. Construction of a genetically stable, transformed fungal strain using restriction enzyme mediated integration (REMI)

In a first aspect, the present disclosure provides a method of constructing a genetically stable, transformed fungal strain comprising: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In the first aspect, the present disclosure also provides a method of constructing a genetically stable, transformed *Trichoderma* strain, said method comprising: a) transforming *Trichoderma* cells using a mixture of linearized (also called "linear") or circular DNA and a restriction enzyme (also called "with treatment" of the restriction enzyme), wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with treatment of the restriction enzyme is higher than percentage of stable transformants obtainable without treatment of the restriction enzyme.

In the first aspect, the present disclosure also provides a method of constructing a genetically stable, transformed *Trichoderma* strain, said method comprising: a) transforming *Trichoderma* cells with linearized or circular DNA, wherein the *Trichoderma* cells are induced to produce a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with induction of the restriction enzyme is higher than percentage of stable transformants obtainable without induction of the restriction enzyme.

In a second aspect, the present disclosure provides a genetically stable, transformed fungal strain obtained using the method as described in the first aspect above.

In a third aspect, the present disclosure provides a method of improving the stability of fungal transformants comprising transforming fungal cells using a mixture of linearized DNA and a restriction enzyme, wherein the restriction enzyme is capable of generating a double strand break in chromosomal DNA of said fungal strain.

In the third aspect, the present disclosure also provides a method of improving the stability of *Trichoderma* transformants comprising transforming *Trichoderma* cells using a mixture of linearized or circular DNA and a restriction enzyme, wherein the restriction enzyme is capable of generating a double strand break (DSB) in the chromosomal DNA of the *Trichoderma* cells, and wherein percentage of resulted stable transformants with treatment of the restriction enzyme is higher than percentage of stable transformants obtainable without treatment of the restriction enzyme.

In the third aspect, the present disclosure also provides a method of improving the stability of *Trichoderma* transformants comprising transforming *Trichoderma* cells with a linearized or circular DNA, wherein the *Trichoderma* cells are induced to produce a restriction enzyme that is capable of generating a double strand break (DSB) in the chromosomal DNA of the *Trichoderma* cells, and wherein percentage of resulted stable transformants with induction of the restriction enzyme is higher than percentage of stable transformants obtainable without induction of the restriction enzyme.

### a. Classical method of fungal strain construction

The use of fungal strains as production hosts for proteins of interest have long been deemed economically viable and widely used in commercial settings for some time. Different genetic methods for transformation and strain construction have been developed for fungi including the standard methods by PEG/protoplast transformation (Hinnen et al., 1978), electrotransformation (Karube et al., 1985), biolistics (Armaleo et al., 1990), *Agrobacterium tumefaciens* mediated transformation (ATMT) (de Groot et al., 1998), and RNA interference (RNAi) techniques (Akashi et al., 2005).

In certain embodiments, to prepare *Aspergillus* sp. or *Hypocrea* sp. *(Trichoderma* sp.) for transformation, protoplasts from fungal mycelium are prepared. See Campbell et al. Curr. Genet. 16:53-56; 1989. Mycelium can be obtained from germinated vegetative spores. The mycelium is treated with an enzyme that digests the cell wall, resulting in protoplasts. The protoplasts are then protected by the presence of an osmotic stabilizer in the suspending medium. Suitable stabilizers include, for example, sorbitol, mannitol, potassium chloride, magnesium sulfate and the like. Typically, the concentration of the stabilizer(s) can vary between about 0.8 M and about 1.2 M (e.g., between about 0.9M and about 1.2 M, between about 1.0M and about 1.2 M, between about 1.1 M and about 1.2 M, etc).

In a particular embodiment, about 1.2 M of sorbitol is used as stabilizer in a suspension medium. Uptake of the DNA into the host strain (e.g., *Aspergillus* sp. or *Hypocrea* sp. *(Trichoderma* sp.) can often be dependent upon the calcium ion concentration. Generally between about 10 mM CaCl₂ and about 50 mM CaCl₂ (e.g., between about 15 mM and about 45 mM, between about 20 mM and about 40 mM, between about 25 mM and about 35 mM) is used in an uptake solution. Aside from including calcium ion in the uptake solution, other items often included are a buffering system such as a TE buffer (10 mM Tris, pH 7.4; 1 mM EDTA) or a 10 mM MOPS, pH 6.0 buffer (morpholinepropanesulfonic acid) and polyethylene glycol (PEG). It is believed that the polyethylene glycol in this buffer acts to fuse the cell membranes thus permitting the contents of the medium to be delivered into the cytoplasm of the host strain (e.g., *Aspergillus* sp. or *Hypocrea* sp), and the plasmid DNA is transferred to the nucleus. In certain embodiments, this fusion process leaves multiple copies of the plasmid DNA integrated into the host chromosome.

Usually a suspension containing the *Aspergillus* sp. protoplasts or cells that have been subjected to a permeability treatment at a density of 10⁵ to 10⁶ /mL, preferably 2 x10 ⁵/mL are used in transformation. Similarly, a suspension containing the *Hypocrea* sp. *(Trichoderma* sp.) protoplasts or cells that have been subjected to a permeability treatment at a density of 10⁸ to 10⁹ /mL, preferably 2 x 10 ⁸/mL are used in transformation. A volume of 100 µL of these protoplasts or cells in an appropriate solution (e.g., 1.2 M sorbitol; 50 mM CaCl2) is mixed with the desired DNA. In some embodiments, a substantial amount of PEG is added to the uptake solution. For example, from about 0.1 to about 1 volume of 25% PEG 4000 can be added to the protoplast suspension. In a particular example, about 0.25 volume of 25% PEG4000 is added to the protoplast suspension. Additives such as dimethyl sulfoxide, heparin, spermidine, potassium chloride and the like may also be added to the uptake solution and aid in transformation.

In certain embodiments, the mixture is incubated at about 0°C, for a period of about 10 to about 30 minutes. Additional PEG can be added to the mixture to further enhance the uptake of the desired gene or DNA sequence. In certain embodiments, the 25% PEG 4000 can be added in volumes that are 5 to 15 times that of the transformation mixture; however, greater and lesser volumes may also be suitable. For example, the 25% PEG 4000 is added at 10 times the volume of the transformation mixture in some embodiments. After the PEG is added, the transformation mixture is then incubated either at room temperature or on ice before the addition of a sorbitol and CaCl₂ solution. The protoplast suspension is then further added to molten aliquots of a growth medium. This growth medium permits the growth of transformants. Many growth media can be suitably used to grow the desired transformants in the present disclosure. In certain embodiments, for example, if Pyr+ transformants are being selected it is preferable to use a growth medium that contains no uridine. For example, the colonies are transferred and purified on a growth medium depleted of uridine.

At this stage, stable transformants may be distinguished from unstable transformants by their faster growth rate. Also, with a number of filamentous fungal hosts, such as, for example, *Trichoderma,* the formation of circular colonies with a smooth, as opposed to a ragged outline on solid culture medium lacking uridine can be used as a distinguishing feature. In some embodiments, further tests and selections of stability may be made by growing the transformants on solid non-selective medium (e.g., containing uridine), harvesting spores from this culture medium, and determining the percentage of these spores. The selected spores are allowed to germinate and grow on selective medium lacking uridine. Percentages of stable transformants can be calculated among all the transformants tested.

But using the above transformation method, the percentage of stable transformants may vary between 5 and 30%, which means a large number of transformants have to be screened to select the best strain. The stability frequency may depend on the selective marker used and in the *Trichoderma* strain. It is still unknown why the frequency of unstable transformants is very high in *Trichoderma* in comparison to some other fungi, like *Aspergilli.* Because of this high level of unstable transformants, the process of screening and selection of production strains tends to be very laborious and time consuming. Therefore, reliable means of producing a genetically stable, transformed fungal strain to express proteins of interest, with reduced variability, provide clear advantages.

When exogenous DNA integrates into the chromosome as targeted gene replacement, it involves the cellular repair mechanisms of DNA DSBs (double strand breaks). DSBs play a critical role in the maintenance of the genome through two main pathways: the HR (homologous recombination) pathway that depends on homologous sequences and the NHEJ pathway that does not depend on sequence homology (Kanaar et al., 1998). These two repair mechanisms are considered to work independently, but reveal competition in function (van Dyck et al., 1999). In the unicellular eukaryote *S. cerevisiae,* HR is the main pathway involved in repairing DSBs, whereas the low frequency (5%) of HR in most filamentous fungi hampered research on targeted gene replacement (Weld et al., 2006). Consequently, a method to increase the HR pathway through elimination of the NHEJ pathway is needed to achieve highly efficient gene targeting in filamentous fungi. NHEJ-deficient strains have been developed to improve the efficiency of HR, because the gene will integrate via HR at its endogenous locus more easily. Although the morphology of NHEJ-deficient strains was similar to wild type, mutants were more sensitive to several chemicals such as phleomycin, bleomycin, and methyl/ethyl methanesulfonate. Therefore, other method, system or technology needs to be developed in order that the gene of interest can be integrated into fungal genome in a targeted fashion.

### b. Fungal strain construction using restriction enzyme mediated integration (REMI)

Restriction enzyme mediated integration (REMI), based on the PEG-protoplast system, was initially established in *S. cerevisiae* (Schiest and Petes, 1991), and has been successfully applied in several fungal phytopathogens, such as *Cochliobolus heterostrophus* (Lu et al., 1994) and *Alternaria alternata* (Tanaka et al., 1999). Today, REMI techniques have been successful for generating higher number or percentage of fungal mutants useful in identification of pathogenicity genes as well as other gene-function assessments. For example, the nematode-trapping fungus *Arthrobotrys oligospora* was treated with optimized REMI methods, resulting in 34-175 transformants per µg linear plasmid DNA, and in total over 2000 transformants. Additionally, 11 interesting mutants screened in the mutant library of *Fusarium graminearum* via REMI and another two genes associated with virulence in the wheat scab, CBL1 and MSY1, were also identified (Seong et al., 2005). Moreover, several proteins related to the pathogenicity or resistance to plant defense compounds were identified from *F. oxysporum* by REMI technique (Duyvesteijn et al., 2005; Imazaki et al., 2007; Madrid et al., 2003). In summary, improved mutant (transformant) frequencies and single-copy insertion rates can be reliably obtained by REMI.

The present disclosure provides the use of REMI in a heretofore unrecognized and unapplied context, that is, REMI was used to significantly increase the percentage of stable *Trichoderma* transformants as opposed to the frequency of transformants. In fact the frequency of transformants was found to be less when REMI was applied to *Trichoderma,* contrary to what would have been expected. However, among the transformants that were made, be it at reduced frequency, the number of stable tranformants dramatically increases.

Described from another perspective, when *Trichoderma* was transformed with DNA in the presence of a restriction enzyme, the number of unstable transformants dropped dramatically, thus allowing for an easy selection of stable colonies (FIGS. 4A-4E).

In a traditional PEG-mediated transformation experiment, the percentage of stable transformants may vary between 5 and 30%. In contrast, when REMI is applied for *Trichoderma* PEG-mediated transformation, the percentage of stable transformants increases up to 90%. As a result the number of transformants that have to be screened to select the best strain can be reduced significantly.

In addition, a higher percentage of high producing transformants was observed when REMI was applied.

In some embodiments, the restriction enzyme used for the method or with the fungal host strains described herein is generated by an expression cassette placed in the fungal host cell. The expression cassette can, for example, suitably be inserted into any expression vector containing a selectable marker and sequences useful for high-level transcription.

It is contemplated that the coding region for restriction enzyme or a part thereof can be inserted into such a general-purpose expression vector, placing it under the transcriptional control of the expression cassettes promoter and terminator sequences. A non-limiting example of such a general-purpose expression vector is pRAX in *Aspergillus.* The gene or variant gene of interest, or a part thereof, can be inserted downstream of the strong *glaA* promoter. A non-limiting example of such a general-purpose expression vector is pTrex3gM in *Hypocrea.* The gene or variant gene of interest, or a part thereof, can be inserted downstream of the strong *cbhl* promoter.

In certain embodiments, in the vector, the DNA sequence encoding the restriction enzyme or protein of interest may be operably linked to transcriptional and translational sequences, for example, a suitable promoter sequence and signal sequence, in reading frame to the structural gene. The promoter is suitably any DNA sequence that shows transcriptional activity in the particular host cell and may be derived from genes encoding proteins either homologous or heterologous to the host cell. An optional signal peptide may provide for extracellular production of the protein or variant of interest. The DNA encoding the signal sequence is preferably that which is naturally associated with the gene to be expressed. However signal sequences from any suitable sources, for example from an exo-cellobiohydrolase or from an endoglucanase of *Trichoderma,* are contemplated.

Protocols that can be used to ligate the DNA sequences coding for restriction enzyme or the protein of interest to a promoter, and insertion of such a construct into suitable vectors are known in the art.

The DNA vector or construct described herein may be introduced into the fungal host cell in accordance with known techniques such as transformation, transfection, microinjection, microporation, biolistic bombardment and the like described above.

The fungal strain that are suitably used may be one from *Trichoderma spp.,* particularly *Trichoderma reesei* (*longibrachiatum*). However, the genes of interest may also be derived from any other suitable microorganisms including fungi, such as *Absidia spp.; Acremonivm spp.; Agancus spp., Anaeromyces spp ; Aspergillus spp, including A. auculeatus, A. awamon, A. flavus, A. foetidus, A. fumaricus, A. fumigatus, A. nidulans, A. niger, A. oryzae, A. terreus and A. versicolor, Aeurobasidium spp.; Cepha*/*osporum spp.; Chaetomium spp.; Chrysosporium spp ; Coprinus spp ; Dactyllum spp.; Fusarium spp., including F. conglomerans, F. decemcellulare, F. javanicum, F. lim, Foxysporum and F. solani; Gliocladium spp.; Humicola spp, including H. insolens and H. lanuginosa; Mucorspp.; Neurospora spp., including N. crassa and N. sitophila; Neocallimastix spp ; Orpinomyces spp.; Penicillium spp; Phanerochaete spp.; Phlebia spp.; Piromyces spp.; Pseudomonas spp.; Rhizopus spp.; Schizophyllum spp.; Trametes spp ; Trichoderma spp.,* including *T reesei, T reesei* (*longibrachiatum*) and *T. vinde; and Zygorhynchus spp.* Similarly, it is envisioned that the gene of interest, or the protein it encodes, may be found in bacteria such as *Bacillus spp., Cellulomonas spp.; Clostridium spp., Myceliophthora spp.; Thermomonospora spp ; Streptomyces spp., S. olivochromogenes; Fibrobacter succinogenes,* and in yeast including *Candida torresn; C. parapsllosis; C. sake; C. zeylanoides, Pichia minuta; Rhodotorula glutinis; R. mucilaginosa,* and *Sporobolomyces roseus.* The gene of interest may alternatively be derived from other sources such as bacteria.

### c. A targeted gene integration system in fungal strain

In one aspect, the present disclosure provides a fungal expression system, comprising, in the first part, a fungal host cell containing at least one restriction enzyme site in chromosomal DNA; and in a second part, a nucleic acid molecule containing a sequence operable to express one or more genes of interest, a selective marker and sequences with substantial homology to sequences that flank said chromosomal restriction enzyme site, wherein the homologous sequences cause a homologous recombination event that results in the expression of the gene of interest. Moreover, the present disclosure provides a method of employing the fungal expression system of the aspect above, comprising transforming said nucleic acid molecule into the fungal host cell in the presence of a restriction enzyme capable of generating the restriction enzyme site.

Furthermore, the present disclosure provides a transformed or derivative fungal strain obtained by the method of employing the fungal expression system.

Yet further, the present disclosure provides a method of targeted gene integration, comprising first obtaining a fungal host cell containing at least one restriction enzyme site in chromosomal DNA, and a nucleic acid molecule containing a sequence operable to express one or more genes of interest, at least one selective marker and sequences with substantial homology to sequences that flank said chromosomal restriction enzyme site, wherein the homologous sequences cause a homologous recombination event that results in the expression of the gene of interest, then transforming the nucleic acid molecule of the first step into the fungal host cell of the first step, in the presence of a restriction enzyme capable of generating the restriction enzyme site.

The instant disclosure provides a targeted gene integration system developed for elucidating gene function including identifying the interacting protein partners of a gene product in both microbial and eukaryotic genomes. Traditionally, methods for determining gene function typically involve the creation of a null mutation for a gene of interest to assess the phenotype of the organism, comparing when the gene of interest is rendered nonfunctional vs. when the gene of interest is. The null mutations are produced by gene disruption (also called gene knockout or gene replacement) using gene disruption vectors made using recombinant DNA techniques. Upon transformation into a relevant organism the DNA constructs with disrupted gene(s) of interest would integrate at the resident location(s) in the genome of such an organism by homologous recombination, replacing the functional copies of the genes. The traditional process as described above has worked well for disruption of genes in a range of organisms but has several inherit limitations. For instance, non-homologous end join (NHEJ) predominating in multicellular eukaryotes, including most of fungi, leads to the expression vectors being integrated into the genome at random. In addition, even among the transformants, large percentages of unstable ones are typically generated, necessitating further screening of the transformants to obtain the stable ones.

In addition, the high-throughput screening for the identification of protein or variants with a higher specific activity using a fungal system, for example, one involving *Trichoderma reesei,* has been a challenge due to the low efficiency of targeted integration events. On the other hand, it is long understood that, in order to provide meaningful screening of proteins/variants/enzymes that can then be expressed or produced by a fungal host organism, which then can be put to industrial uses, it is critical to conduct such high-throughput screening in the fungal system in which the protein/variant/enzyme of interest will eventually be produced, or at least a fungal system that is relatively closely related. However, in fungal systems such as the *Trichoderma* system, favored by many skilled in the art, random integration of an expression cassette containing the mutated gene of interest is nearly inevitable and frequently occurs, giving rise to two variables, the interplay of which leads to extraordinary complexities: 1) the expression level; and 2) the activity of the protein/variant/enzyme of interest. Accordingly, approaches such as the one described herein, which result in homogeneity in protein expression of transformants via targeted integration of a gene of interest to a predetermined site in the genome, are highly desirable as protein activities can be directly related the characteristic mutation.

Previously efforts have been made to reduce the variability of the expression level. For example, mutant fungal strains, in which the non-homologous-end-joining (NHEJ) pathway (e.g. ku70, ku80 or lig4) has been inactivate, have been found to display increases frequencies of homologous integration. Unfortunately, the number of transformants thus obtained was quite low. In addition, mutations in ku70 in industrial strains have been reported to be undesirable at times, when they led to genetic instability (Aw and Polizzi 2013).

The present disclosure demonstrates that, by using *I-Sce*I endonuclease to create a double strand break at a predetermined site in the *T. reesei* genome, the targeted integration frequency was improved with NHEJ genes intact in *T. reesei* strain.

In a particular example (Example 2), a recombinant *T. reesei* was constructed to harbor the *I-Sce*I restriction sites at the cbh2 locus, and a glucoamylase expression cassette with substantial homology to sequences that flank *I-SceI* restriction sites was used to transform the recombinant *T. reesei* in the presence of *I-SceI,* which created a double strand break at a predetermined site in the *T. reesei* genome. Targeted integration of the glucoamylase expression cassette at the intended locus via a double cross over was observed (FIG. 11). As further demonstrated in Table 3, 68% of transformants (*I-SceI* induced) expressing glucoamylase were integrated in a targeted fashion, as versus a mere 46% for a non-induced *I-SceI* construct, and 16% for the control construct.

### d. Fermentation of the fungal strain

In one aspect, the present disclosure provides a method of fermenting, in a suitable medium, a transformed fungal strain, which has been obtained using the method comprising: a) transforming the cells of a fungus with a mixture of linearized DNA and a restriction enzyme, wherein the restriction enzyme is capable of generating a double strand break in chromosomal DNA of the fungal strain; and b) selecting for a genetically stable, transformed fungal strain produced in step a).

In this aspect, the present disclosure also provides a method of fermenting, in a suitable medium, a transformed fungal strain, which has been obtained using the method comprising: a) transforming *Trichoderma* cells using a mixture of linearized/linear or circular DNA and a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with treatment of the restriction enzyme is higher than percentage of stable transformants obtainable without treatment of the restriction enzyme.

In the same aspect, the present disclosure also provides a method of fermenting, in a suitable medium, a transformed fungal strain, which has been obtained using the method comprising: a) transforming *Trichoderma* cells with linearized/linear or circular DNA, wherein the *Trichoderma* cells are induced to produce a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with induction of the restriction enzyme is higher than percentage of stable transformants obtainable without induction of the restriction enzyme.

In another aspect, the present disclosure provides a method of expressing a gene of interest comprising growing and fermenting a transformed fungal strain, obtained, for example, in accordance with what has been described in the aspect above, in a suitable medium under conditions that allow expression of the gene of interest.

In a further aspect, the present disclosure provides a method of fermenting the transformed or derivative fungal strain in a suitable medium, whereby the transformed or derivative fungal strain is obtained using the methods and fungal expression systems as described herein.

In yet a further aspect, the present disclosure provides a method of expressing a gene of interest comprising growing and fermenting the transformed or derivative fungal strain as provided herein in a suitable medium and under conditions that allow expression of the gene of interest. The transformed or derivative fungal strain thus obtained may suitably employ the fungal expression system as described or provided herein, comprising, generally, transforming a certain nucleic acid molecule into a fungal host cell in the presence of a restriction enzyme capable of producing a suitable restriction enzyme site.

Any of the fermentation methods well known in the art can be suitably used to ferment the transformed or the derivative fungal strain as provided herein. In some embodiments, fungal cells are grown under batch or continuous fermentation conditions.

A classical batch fermentation is a closed system, where the composition of the medium is set at the beginning of the fermentation, and the composition is not altered during the fermentation. At the beginning of the fermentation, the medium is inoculated with the desired organism(s). In other words, the entire fermentation process takes place without addition of any components to the fermentation system throughout.

Alternatively, a batch fermentation qualifies as a "batch" with respect to the addition of the carbon source. Moreover, attempts are often made to control factors such as pH and oxygen concentration throughout the fermentation process. Typically the metabolite and biomass compositions of the batch system change constantly up to the time the fermentation is stopped. Within batch cultures, cells progress through a static lag phase to a high growth log phase and finally to a stationary phase, where growth rate is diminished or halted. Left untreated, cells in the stationary phase would eventually die. In general, cells in log phase are responsible for the bulk of production of product.

A suitable variation on the standard batch system is the "fed-batch fermentation" system. In this variation of a typical batch system, the substrate is added in increments as the fermentation progresses. Fed-batch systems are useful when it is known that catabolite repression would inhibit the metabolism of the cells, and/or where it is desirable to have limited amounts of substrates in the fermentation medium. Measurement of the actual substrate concentration in fed-batch systems is difficult and is therefore estimated on the basis of the changes of measurable factors, such as pH, dissolved oxygen and the partial pressure of waste gases, such as CO₂. Batch and fed-batch fermentations are well known in the art.

Continuous fermentation is another known method of fermentation. It is an open system where a defined fermentation medium is added continuously to a bioreactor, and an equal amount of conditioned medium is removed simultaneously for processing. Continuous fermentation generally maintains the cultures at a constant density, where cells are maintained primarily in log phase growth. Continuous fermentation allows for the modulation of one or more factors that affect cell growth and/or product concentration. For example, a limiting nutrient, such as the carbon source or nitrogen source, can be maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

### e. Proteins of interest produced by the fungal strain

In one aspect, the present disclosure provides a protein of interest encoded by a gene of interest and produced by a method of expressing a gene of interest comprising growing and fermenting a transformed fungal strain in a suitable medium under conditions that allow expression of the gene of interest, which strain is obtained using the method comprising: a) transforming fungal cells using a mixture of linearized DNA and a restriction enzyme, wherein the restriction enzyme is capable of generating a double strand break in chromosomal DNA of said fungal strain; and b) selecting a genetically stable, transformed fungal strain produced in step a).

In this aspect, the present disclosure also provides a protein of interest encoded by a gene of interest and produced by a method of expressing a gene of interest comprising growing and fermenting a transformed fungal strain in a suitable medium under conditions that allow expression of the gene of interest, which strain is obtained using the method comprising: a) transforming *Trichoderma* cells using a mixture of linearized/linear or circular DNA and a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with treatment of the restriction enzyme is higher than percentage of stable transformants obtainable without treatment of the restriction enzyme.

In the same aspect, the present disclosure also provides a protein of interest encoded by a gene of interest and produced by a method of expressing a gene of interest comprising growing and fermenting a transformed fungal strain in a suitable medium under conditions that allow expression of the gene of interest, which strain is obtained using the method comprising: a) transforming *Trichoderma* cells with linearized/linear or circular DNA, wherein the *Trichoderma* cells are induced to produce a restriction enzyme, wherein the restriction enzyme is capable of generating a double strand break (DSB) in the chromosomal DNA of the *Trichoderma* cells; and b) selecting a genetically stable, transformed *Trichoderma* strain produced in a), wherein percentage of stable transformants with induction of the restriction enzyme is higher than percentage of stable transformants obtainable without induction of the restriction enzyme.

In another aspect, the present disclosure provides a protein of interest encoded by a gene of interest and produced by a method of expressing a gene of interest in a derivative fungal strain, in a suitable medium under conditions that allow expression of the gene of interest, which strain is obtained by the method employing a fungal expression system comprising, in a first part, a fungal host cell containing at least one restriction enzyme site in chromosomal DNA; and in a second part, a nucleic acid molecule containing a sequence operable to express one or more genes of interest, a selective marker and sequences with substantial homology to sequences that flank said chromosomal restriction enzyme site, wherein the homologous sequences cause a homologous recombination event that results in the expression of the gene of interest, whereby the nucleic acid molecule of the first part is transformed into the fungal host cell in the presence of a restriction enzyme capable of producing the restriction enzyme site.

The protein of interest can suitably be any endogenous and heterologous protein. The protein of interest can contain one or more disulfide bridges or may be a protein whose functional form is a monomer or multimer, i.e. the protein has a quaternary structure and is composed of a plurality of identical (homologous) or non-identical (heterologous) subunits, however, the protein of interest is preferably the protein with properties of interest.

The protein of interest may be a variant of interest. The protein of interest or the variant of interest may suitably be a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, or a variant thereof, or a mixture of two or more of these enzymes or variants. Non-limiting examples of proteins of interest or variant protein of interests may be proteins or enzymes, or variants thereof, involved in starch metabolism, proteins or enzymes or variants thereof, involved in glycogen metabolism, enzymes such as acetyl esterases, aminopeptidases, amylases, arabinases, arabinofuranosidases, carboxypeptidases, catalases, cellulases, chitinases, chymosin, cutinase, deoxyribonucleases, epimerases, esterases, α-galactosidases, β-galactosidases, α-glucanases, glucan lysases, endo- β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, peroxidases, phenoloxidases, phytases, polygalacturonases, proteases, rhamno-galacturonases, ribonucleases, thaumatin, transferases, transport proteins, transglutaminases, xylanases, hexose oxidase (D-hexose: 02- oxidoreductase, EC 1.1.3.5), variants thereof, and combinations or two or more of these enzymes or variants thereof.

The protein of interest or the variant of interest may also suitably be a peptide hormone, a growth factor, a clotting factor, a chemokine, a cytokine, a lymphokine, an antibody, a receptor, an adhesion molecule, a microbial antigen (e.g., HBV surface antigen, HPV E7, etc.), or variants or fragment thereof, or a mixture of two or more of such substances, variants or fragments.

Other types of proteins or variants of interest may be those that are capable of providing nutritional value to a food or to a crop. Non-limiting examples include plant proteins that can inhibit the formation of anti-nutritive factors and plant proteins that have a more desirable amino acid composition (e.g. a higher lysine content than a non-transgenic plant).

### f. Use of the proteinaceous composition thus made

In one aspect, the present disclosure provides a proteinaceous composition comprising the protein of interest. The proteinaceous composition is suitably produced using a method provided herein. The composition comprises a protein of interest, encoded by a gene of interest, expressed using a method described herein. The composition may be used in various useful industrial applications such as, for example, in biomass hydrolysis, cleaning applications, grain processing, animal nutrition, food composition, textile treatment, and the like.

For example, the proteinaceous composition thus produced can be used in lignocellulosic biomass hydrolysis. Lignocellulose, the world's largest renewable biomass resource, is composed mainly of lignin, cellulose, and hemicellulose, of which the large part of the latter is xylan. The conversion of lignocellulosic feedstocks into ethanol has the advantages of the ready availability of large amounts of feedstock, the desirability of avoiding burning or land filling the materials, and the cleanliness of the ethanol fuel. Wood, agricultural residues, herbaceous crops, and municipal solid wastes have been considered as feedstocks for ethanol production. Once the lignocellulose is converted to fermentable sugars, e.g., glucose, the fermentable sugars are easily fermented by yeast into ethanol. Cellulose is a polymer of the simple sugar glucose covalently linked by beta-1,4-bonds. Many microorganisms produce enzymes that hydrolyze beta-linked glucans. These enzymes include endoglucanases, cellobiohydrolases, and beta-glucosidases. Xylans are polysaccharides formed from 1,4-β-glycoside-linked D-xylopyranoses. Xylanases (e.g., endo-1,4-beta-xylanase, EC 3.2.1.8) hydrolyze internal β-1,4-xylosidic linkages in xylan to produce smaller molecular weight xylose and xylo-oligomers. The disclosure provides a transformed fungal cell, which has demonstrated improved transformant stability, suitable and advantageous as a producer of industrial enzymes, variants, and mixtures of interest to such lignocellulosic biomass use.

In another example, the proteinaceous composition thus produced can be used in cleaning application. Enzymatic cleaning components are popular because of their ability to break down soils, stains, and other debris that are otherwise not readily removed by conventional chemical detergents. Well-known enzymes useful for cleaning include proteases and amylases, with other enzymes such as lipases, pectinases, mannanases, even certain cellulases, each providing a set of different functionalities. Proteases combat protein-based stains; amylases work on carbohydrates and starches; and lipases break down lipids or fats, for example. The disclosure provides a transformed fungal cell, which has demonstrated improved transformant stability, suitable and advantageous as a producer of industrial enzymes, variants, and mixtures of interest to such use in cleaning applications.

In another example, the proteinaceous composition thus made can be used in grain procession. Starch is the commonest storage carbohydrate in plants, used by the plants themselves as well as by microbes and by higher organisms. A great variety of enzymes are able to catalyze starch hydrolysis. Starch from all plant sources occurs in the form of granules, but depending on the species of the plant source, starch presents in markedly different size and physical characteristics. Acid hydrolysis of starch had widespread use in the past, however this process has now largely been replaced by enzymatic processes, which are known to demand less corrosion-resistant materials and other benefits, need less energy for heating and are relatively easier to control than the acid process. The disclosure provides a transformed fungal cell, which has demonstrated improved transformant stability, suitable and advantageous as a producer of industrial enzymes, variants, and mixtures of interest to such use in starch degradation and grain processing.

In another example, the proteinaceous composition thus made can be used in food application. Enzymes produced by bacteria, yeasts and moulds have been used in food application to make foods such as bread, cheese, beer and wine for many thousands of years. Today enzymes are used in bakery, cheese making, starch processing and production of fruit juices and other drinks, providing various benefits such improved texture, appearance and nutritional value, generate desirable flavors and aromas, and the like. Food enzymes typically originate in animals and plants (for example, a starch-digesting enzyme, amylase, can be obtained from germinating barley seeds) as well as from a range of beneficial microorganisms. Enzymes are deemed viable and desirable alternatives to traditional chemical-based technology, replacing synthetic chemicals in many processes. Enzymes can help improve the environmental performance of food production processes, reducing energy consumption and improving biodegradability of waste or side products. Enzymes tend to be more specific in their actions than synthetic chemicals, and as such, enzymatic processes tend to give fewer side reactions and waste or byproducts, and consequently producing higher quality products and reducing the likelihood of pollution. Enzymatic processes are often also the only processes possible. An example of this is in the production of clear apple juice concentrate, which relies on the use of the enzyme, pectinase. Most of the food enzymes are produced from microorganisms such *Bacillus, Aspergillus, Streptomyces* or *Kluyveromyces.* The disclosure provides a transformed fungal cell, which has demonstrated improved transformant stability, suitable and advantageous as a producer of industrial enzymes, variants, and mixtures of interest to such use in food applications.

In another example, the proteinaceous composition thus made can be used in animal feed additive. Cellulase, xylanase, beta-glucanase, alpha-amylase, protease, lipase, phytase and other carbohydrase have been widely used in animal feed industry. Since many plant based feeds contain substances with anti-nutritional factors that reduce animal growth, the enzymes added to such feeds improve digestibility of these anti-nutritional factors by degrading fibres, proteins, starches and phytates, rendering them more digestible by the animals, and enabling the use of cheaper and often locally produced feeds, while maximizing meat, egg or milk productivity. At the same time, the enzymes added to such feeds also may provide benefits supporting gut health and enhanced animal performance. The disclosure provides a transformed fungal cell, which has demonstrated improved transformant stability, suitable and advantageous as a producer of industrial enzymes, variants, and mixtures of interest to such use in animal feed applications.

In yet a further example, the proteinaceous composition thus made can be used in textile applications. Enzymes have become an integral part of the textile processing. There are two well-established enzyme applications in the textile industry. First, enzymes such as amylases are commonly used in the preparatory finishing area for desizing. Second, enzymes such as cellulases are commonly used in the finishing area for softening, bio-stoning and reducing of pilling propensity of cotton goods. Other enzymes such as, for example, pectinases, lipases, proteases, catalases, xylanases etc., are also used in textile processing. Moreover, there are various applications which entail enzymes included fading of denim and non-denim, bio-scouring, bio-polishing, wool finishing, peroxide removal, de-colorization of dyestuff, etc. (Cavaco-Paulo and Gubitz, 2003; Chelikani et al., 2004; Nalankilli, 1998; Shenai, 1990). The disclosure provides a transformed fungal cell, which has demonstrated improved transformant stability, suitable and advantageous as a producer of industrial enzymes, variants, and mixtures of interest to such use in textiles applications.

### EXAMPLES

Aspects of the present strains, compositions and methods may be further understood in light of the following examples, which should not be construed as limiting. Modifications to materials and methods will be apparent to those skilled in the art.

A summary of some fungal (*Trichoderma*) strains and plasmids used in this disclosure (mostly constructed in this study) is shown below in Table 1:

| **Fungal strains** | **Genotype or description** |
|---|---|
| QM6a | Wild type *T. reesei* strain (Toyama, 1969) |
| P37ΔcbhIpyrG-26 | pyrG- and Pcbh1- |
| JP7.7 | p37Δcbh1pyrG-26 with I-SceI restriction sites cassette integrated at cbh2 locus |
| JP7.7.12 | JP7.7+pTTT-ISceI |
| JP7.7.14 | JP7.7+pTTT-IScel |
| JP7.7.16 | JP7.7+PTTT-ISceI |
| | |

| **Plasmids** | |
|---|---|
| pBJP6 | Carries I-SceI restriction site cassette |
| pCRpyrGAN | Containing the full gene of A. nidulan pyrG |
| pTTT | cbhI promoter and amdS selection |
| pTTT-Iscel | I-SceI under control of the inducible cbh1 promoter with amdS selection |
| PTrex6gGA/wt | Carries *T. reesei* Glucoamylase gene |
| pJP8 | Carries *T. reesei* glucoamylase casette with homologous regions of the I-SceI landing sites cassette (pBJP6) |

### EXAMPLE 1: AclAmy1 expression using REMI

### A. CONSTRUCTING AclAmy1 EXPRESSION VECTORS

### 1) pTrex3gM-AclAmy1

The enzyme AclAmy1 used here as a model protein of interest refers to an acid stable fungal alpha amylase originating from *Aspergillus clavatus* having the amino acid sequence of (with the signal sequence bolded and italicized) (SEQ ID NO:1):

MKLLALTTAFALLGKGVFGLTPAEWRGQSIYFLITDRFARTDGSTTAPCDLSQRAYCG
GSWQGIIKQLDYIQGMGFTAIWITPITEQIPQDTAEGSAFHGYWQKDIYNVNSHFGTA
DDIRALSKALHDRGMYLMIDVVANHMGYNGPGASTDFSTFTPFNSASYFHSYCPINN
YNDQSQVENCWLGDNTVALADLYTQHSDVRNIWYSWIKEIVGNYSADGLRIDTVKH
VEKDFWTGYTQAAGVYTVGEVLDGDPAYTCPYQGYVDGVLNYPIYYPLLRAFESSS
GSMGDLYNMINSVASDCKDPTVLGSFIENHDNPRFASYTKDMSQAKAVISYVILSDGI
PIIYSGQEQHYSGGNDPYNREAIWLSGYSTTSELYKFIATTNKIRQLAISKDSSYLTSRN
NPFYTDSNTIAMRKGSGGSQVITVLSNSGSNGGSYTLNLGNSGYSSGANLVEVYTCS
SVTVGSDGKIPVPMASGLPRVLVPASWMSGSGLCGSSSTTTLVTATTTPTGSSSSTTLA
TAVTTPTGSCKTATTVPVVLEESVRTSYGENIFISGSIPQLGSWNPDKAVALSSSQYTSS

NPLWAVTLDLPVGTSFEYKFLKKEQNGGVAWENDPNRSYTVPEACAGTSQKVDSSW
R

The nucleotide sequence of the *AclAmy1* gene comprises eight introns, which sequence is presented below (SEQ ID NO:2):

ATGAAGCTTCTAGCTTTGACAACTGCCTTCGCCCTGTTGGGCAAAGGGGTATTTG
GTCTAACTCCGGCCGAATGGCGGGGCCAGTCTATCTACTTCCTGATAACGGACCG
GTTTGCTCGTACAGATGGCTCAACAACCGCTCCATGTGATCTCAGCCAGAGGTT
AGTGATTTCATCGTATTCTTTGTCATGTGTCATGACGCTGACGATTTCAGGCGTAC
TGTGGTGGAAGCTGGCAGGGTATTATCAAGCAAGTAAGCCTACTGGTTTCCAATT
TTGTTGAATTCCTTTCTGACTCGGCCAGCTCGATTATATCCAAGGAATGGGCTTCA
CTGCTATTTGGATCACACCCATTACGGAGCAAATCCCACAGGATACCGCTGAAGG
ATCAGCATTCCACGGCTATTGGCAGAAGGATATGTGAGTTTCCTTATAACATTCA
CTACGTTTTGCTAATATAGAACAGTTACAATGTCAACTCCCATTTCGGAACCGCC
GATGACATTCGGGCATTGTCCAAGGCCCTTCACGACAGGGGAATGTACCTGATG
ATTGACGTTGTTGCCAACCACATGGTAGGTGATATCTCACTGATTGAGTTATACC
ATTCCTACTGACAGCCCGACCTCAACAAAAGGGTTACAATGGACCTGGTGCCTCG
ACTGATTTTAGCACCTTTACCCCGTTCAACTCTGCCTCCTACTTCCACTCGTACTG
CCCGATCAACAACTATAACGACCAGTCTCAGGTAGAGAACTGTTGGTTGGGAGA
CAACACTGTGGCTCTGGCAGACCTATACACCCAGCATTCGGATGTGCGGAACATC
TGGTACAGCTGGATCAAAGAAATTGTTGGCAATTACTCTGGTTAGTAATCCAATC
CAAGTCCCGTCCCCTGGCGTCTTTCAGAACTAACAGAAACAGCTGATGGTCTGCG
TATCGACACCGTCAAGCACGTTGAAAAGGATTTCTGGACTGGCTACACCCAAGCT
GCTGGTGTTTATACCGTTGGCGAGGTATTAGATGGGGACCCGGCTTATACCTGCC
CCTATCAGGGATATGTGGACGGTGTCCTGAATTATCCCATGTGAGTTCACCCTTT
CATATACAGATTGATGTACTAACCAATCAGCTATTATCCCCTCCTGAGAGCGTTC
GAATCGTCGAGTGGTAGCATGGGTGATCTTTACAATATGATCAACTCTGTGGCCT
CGGATTGTAAAGACCCCACCGTGCTAGGAAGTTTCATTGAGAACCATGACAATCC
TCGCTTCGCTAGGTAGGCCAATACTGACATAGGAAAGGAGAAGAGGCTAACTGT
TGCAGCTATACCAAGGATATGTCCCAGGCCAAGGCTGTTATTAGCTATGTCATAC
TATCGGACGGAATCCCCATCATCTATTCTGGACAGGAGCAGCACTACTCTGGTGG
AAATGACCCGTACAACCGCGAAGCTATCTGGTTGTCGGGTTACTCTACCACCTCA
GAGCTGTATAAATTCATTGCCACCACGAACAAGATCCGTCAGCTCGCCATTTCAA
AGGATTCAAGCTATCTTACTTCACGAGTATGTGTTCTGGCCAGACTCACACTGCA

ATACTAACCGGTATAGAACAATCCCTTCTACACTGATAGCAACACCATTGCAATG
CGAAAGGGCTCCGGGGGCTCGCAGGTCATCACTGTACTTTCCAACTCTGGTTCCA
ACGGTGGATCGTACACGCTCAACTTGGGTAACAGCGGATACTCGTCTGGAGCCA
ATCTAGTGGAGGTGTACACCTGCTCGTCTGTCACGGTCGGTTCCGACGGCAAGAT
CCCCGTCCCCATGGCATCTGGTCTTCCCCGTGTCCTTGTTCCGGCATCTTGGATGT
CCGGAAGTGGATTGTGCGGCAGCTCTTCCACCACTACCCTCGTCACCGCCACCAC
GACTCCAACTGGCAGCTCTTCCAGCACTACCCTCGCCACCGCCGTCACGACTCCA
ACTGGTAGCTGCAAAACTGCGACGACCGTTCCAGTGGTCCTTGAAGAGAGCGTG
AGAACATCCTACGGCGAGAACATCTTCATCTCCGGCTCCATCCCTCAGCTCGGTA
GCTGGAACCCGGATAAAGCAGTCGCTCTTTCTTCCAGCCAGTACACTTCGTCGAA
TCCTTTGTGGGCCGTCACTCTCGACCTCCCCGTGGGAACTTCGTTTGAATACAAAT
TCCTCAAGAAGGAGCAGAATGGTGGCGTCGCTTGGGAGAATGACCCTAACCGGT
CTTACACTGTTCCCGAAGCGTGTGCCGGTACCTCCCAAAAGGTGGACAGCTCTTG
GAGGTGA

This protein has a calculated mass of 67 kilo Dalton (kDa), and is, like others in the class EC 3.2.1.1., capable of hydrolyzing alpha-bonds of large alpha-linked polysaccharides (e.g. starch). The optimal pH is 4.5, as measured on PAHBAH (*p*-Hydroxy benzoic acid hydrazide) as substrate, while high enzyme activity is found at the broad range of pH 3-7.

The temperature where its enzyme activity is optimal is 66 °C.

A pTrex3gM-AclAmy1 vector was designed to drive the expression of AclAmy1 from its native coding sequence, which was placed under the control of a strong *cbh*I promoter. The *AclAmy1* gene encoding AclAmy1 was amplified from the *Aspergillus clavatus* chromosome with PCR using the following primers:
Primer 1 (5'- ggggcggccgccaccATGAAGCTTCTAGCTTTGACAAC-3') (SEQ ID NO:3); and
Primer 2 (5'-cccggcgcgccttaTCACCTCCAAGAGCTGTCCAC-3') (SEQ ID NO:4).

After digestion with restriction enzymes *Not*I and *Asc*I, the PCR product was cloned into pTrex3gM expression vector (as described in U.S. Published Application 2011/0136197 A1), digested with the same restriction enzymes, and the resulting plasmid was labeled pTrex3gM-AclAmy1. A plasmid map of pTrex3gM-AclAmy1 is provided in FIG. 1. A complete list of functional elements of the plasmid is also provided in FIG. 1.

The sequence of the *AclAmy1* gene was confirmed by DNA sequencing.

### 2. pENTRY-AclAmy1

To construct an expression vector pTrex8gM-AclAmyl1, the *A. clavatus* Amy1 encoding fragment was first recloned from the plasmid pTrex3gM-AclAmy1 (as described above) into the pDonor221 vector via a Gateway® BP reaction according to the recommendation of the supplier (Life Technologies, Carlsbad CA). The resulting pEntry-AclAmy1 plasmid and its functional elements are shown in FIG. 2.

### 3. pTrex8gM-AclAmy1

The ENTRY vector DNA fragment encoding the *A. clavatus* alpha amylase was further transferred into pTREX8gM via a Gateway® LR reaction resulting in a plasmid pTREX8gM-AclAmy1 (FIG. 3). Functional elements of pTrex8gM-AclAmy1 are listed in FIG. 3.

Clones were verified by sequencing the coding regions.

### B. TRANSFORMATION FOR ECTOPIC EXPRESSION

### 1. Protoplast Preparation

Spores of a *Trichoderma* strain were inoculated into 50 mL of YEG culture medium (5 g/L yeast extract, 20 g/L glucose) and grown in a 250 mL shake flask overnight at 28 °C, at a rate of 180 rpm in a shaker incubator (Infors-HT, Switzerland) with a 50 mm throw. In some experiments, a derivative of *T. reesei* strain RL-P37 with deletions of cbh1, cbh2, egl1 and egl2 genes was used, in some other experiments, other *T. reesei* strains were used. Any suitable *Trichoderma* strains would work for experiments like those disclosed herein.

Germinated spores were collected by a 10 min centrifugation (3000 *g*) and washed twice with 10 mL of 1.2 MgSO₄, 10 mM Na-phosphate, at pH 5.8. Pellet was re-suspended in 40 mL of the same buffer supplemented with 1.2 g of lysing enzymes (Sigma, St Louis MO).

This was incubated at 28 °C in a shaker incubator, shaking at a rate of 100-200 rpm until protoplasts are formed.

Suspension was filtered through Miracloth to remove mycelium and an equal volume of 0.6 M sorbitol, 0.1 M Tris-HCl, at pH 7.0, was gently added on top of the protoplast solution. This was centrifuged at 4000 rpm for 15 min.

Protoplasts were found in the interphase, which were then carefully collected and transferred to a new tube. This step was repeated as required until the necessary protoplasts were collected. An equal volume of 1.2 M sorbitol, 10 mM CaCl₂, 10 mM Tris-HCl, at pH 7.5, was added to the protoplasts. The protoplasts were pelleted at 4000 rpm in 15 min, at 4 °C, and washed twice in 1.2 M sorbitol, 10 mM CaCl₂, 10 mM Tris-HCl, at pH 7.5. Finally, protoplasts were re-suspended in the same buffer to a concentration of 1x10⁸ protoplasts/mL and per 200 µL of protoplasts 50 µL of 25% PEG 6000, 50 mM CaCl₂, 10 mM Tris-HCl, at pH 7.5, was added. The resulting material was then stored at -80 °C.

### 2. Transformation for ectopic expression

In some experiments, PCR products were directly used to transform the protoplasts. About 5-20 µg of DNA (the PCR products) was added to 200 µL of protoplasts and incubated on ice for 20 min. After that, transformation mixtures were transferred to room temperature and 2 mL of 25% PEG 6000, CaCl₂, 10 mM Tris-HCl, at pH 7.5, and 4 mL of 1.2 M sorbitol, 10 mM CaCl₂, 10 mM Tris-HCl, at pH 7.5, was added.

If restriction enzyme mediated integration (REMI) was applied to a sample, then 20 units of a restriction endonuclease were added to the DNA of that sample.

In some other experiments, a plasmid DNA was used to transform the *Trichoderma* protoplasts, and in some cases the plasmid DNA was linearized using a restriction endonuclease. After incubating the digestion mixture, the plasmid DNA was not purified from the restriction endonuclease.

In one experiment, a PCR fragment encompassing the region from the *cbhI* promoter through AclAmy1 coding region to the 3' part of pyr2 from the plasmid pTrex8gM-AclAmy1 was obtained using the following primers:
Primer 1: 5'GAGTTGTGAAGTCGGTAATCCCGCTG (SEQ ID NO:5); and
Primer 2: 3'CGATACACGCACCAGGTACCCCAGTGGGGAAGC) (SEQ ID NO:6).

The *PcbhI-AclAmy1-pyr2* PCR product_was used to transform *T. reesei* protoplasts either without addition of any restriction enzyme or with the *Pac*I restriction enzyme. Transformants were selected for uridine prototrophy on AmdS medium supplemented with 10 mM NH₄Cl. For making this medium, a 2X AmdS solution (30 g/L KH₂PO4, 20 mM Acetamide, 1.2 g/L MgSO₄*7H₂O, 1.2 g/L CaCl₂*2H₂O, 0.48 g/L citric acid*H₂O, 0.5 g/L FeSO₄*7H₂O, 40 mg/L ZnSO₄*7H₂O, 8 mg/L CuSO₄.5H₂O, 3.5 mg/L MnSO₄.H₂O, 2 mg/L H₃BO₃ (Boric Acid), 40 g/L glucose, at pH 4.5) was mixed with an equal volume of 4% agar containing 2 M sorbitol.

An example of *Trichoderma* transformation is shown in FIGS. 4A-4E. FIGS. 4A, 4C and 4D show the pictures and percentages of *T. reesei* transformants in a typical transformation experiment without REMI--around 10-20% transformants are morphologically stable. FIGS. 4B and 4E show the pictures and percentages of *T. reesei* transformants in a typical transformation experiment where REMI was applied--around 80-90% transformants are morphologically stable

### 3. Transformants Selection

After growth in a glucose/sophorose production medium, fermentation samples were analyzed for expression levels of *A*. *clavatus* alpha amylase using activity assays with an α-amylase activity kit from Megazyme, UK ("Ceralpha Method").

In parallel, expression was confirmed by SDS-PAGE analysis (Life Technologies, Carlsbad CA).

### C. TRICHODERMA FERMENTATION AND AMYLASE ACTIVITY ASSAYS

*Trichoderma* transformants from the above described derivative of *T. reesei* strain RL-P37 were inoculated in 24-well plates configured such to release lactose from a solid, porous matrix. Each well contained 1.25 mL of an NREL medium (9 g/L casamino acids, 5 g/L (NH₄)₂SO₄, 4.5 g/L KH₂PO4, 1 g/L MgSO₄*7H₂O, 1 g/L CaCl₂*2H₂O, 33 g/L PIPPS buffer, at pH 5.5, 0.25% *T. reesei* trace elements (100%: 175 g/L citric acid (anhydrous), 200 g/L FeSO₄*7H₂O, 16 g/L ZnSO₄*7H₂O, 3.2 g/L CuSO₄.5H₂O, 1.4 g/L MnSO₄.H₂O, 0.8 g/L H₃BO₃ (Boric Acid), 16 g/L glucose). Cleared supernatant was analyzed for α-amylase activity using Ceralpha Method (Megazyme, UK).

Ceralpha is blocked *p*-Nitrophenyl α,-D-maltoheptaoside, plus excess α-glucosidase and glucoamylase for the measurement of cereal, fungal and bacterial α-amylase. Cleared supernatant was diluted in 36 mM sodium acetate buffer, at pH 4.5, by 100-500 fold (this would be dependent on the expected α-amylase activity).

Alternatively, cleared supernatant was diluted in a solution of 10 mM NaCl, 0.1 mM CaCl₂, 0.005% Tween 80, by 100-500 fold (this would again be dependent on the depending on the expected α-amylase activity). Forty (40) µL of diluted cleared supernatant was mixed with an equal volume of Ceralpha substrate and incubated at 28 °C for 10 min. Reaction was stopped by adding 100 µL of a 200 mM sodium borate solution, at pH 10.2. Absorbance was measured at 405 nM and corrected for the dilution factor and expressed as α-amylase activity per minute. Cleared supernatants were also analyzed by SDS-PAGE (Life Technologies, Carlsbad CA).

FIG. 5 shows the difference in α-amylase production level. Stable *Trichoderma* iRNAxyn1,3 transformants, obtained in the presence of active *Pac*I enzyme showed both higher absolute levels of α-amylase activity and higher number of stable transformants with elevated expression of α-amylase. REMI increased the percentage of stable transformants (as compared to unstable ones) and the stable transformants also produced higher titers of α-amylase.

These results were confirmed with a transformation experiment of *T. reesei* iRNAxyn1,3 using another plasmid pTrex8gM AGL2 (expressing an α-galactosidase) in the presence of the restriction enzyme *Ssp*I*.*

As soon as the restriction enzyme was added, the background of unstable transformants decreased significantly and the percentage of stable, α-galactosidase producing transformants increased.

### EXAMPLE 2: THE USE OF I-SCEI TO IMPROVE TARGETED INTEGRATION IN T. REESEI

### A. CONSTRUCTION OF REPORTER STRAIN TO MEASURE I-SCEI ACTIVITY IN T. REESEI

### 1. Construction of I-SceI Landing Sites Cassette: Method

Plasmid *pBJP6* with the *I-SceI* landing sites was designed and constructed as follows and as shown in FIG. 6.

The 5'UTR *cbh2, Pcbh1* and *Tcbh2* were amplified from genomic DNA of *T. reesei* strain QM6A (publically available from various sources, including, without limitation, http://www.uniprot.org/taxonomy/431241) with primers GSP1 and GSP2, PP1 and PP2, GSP3 and GSP4 respectively (Table 2) and the listing herein below. The PCR fragments were fused using phusion DNA polymerase (Fermentas) and cloned into *Xba*I and *Kpn*I restriction sites of pBluescriptSK(+) to generate the plasmid *pBJP4* (FIG. 6).

In addition, another plasmid pGFPrep was constructed to carry a DNA fragment (a *GFPn*-GFPc** construct) encoding a C-terminally truncated GFP (*GFPn* or GFPΔC*) and a DNA fragment encoding an N-terminally truncated GFP (*GFPc* or ΔNGFP*) with 600-bp overlapping GFP sequences (the hatched bar regions). The *GFPn*-GFPc** construct was designed based on the GFP sequence of *Ptilosarcus sp.* and was synthetically ordered from Geneart, Germany. The *GFPn*-GFPc** construct was then cloned into the *Nde*I restriction site of *pBJP4,* between the *cbhI* promoter and *cbhI* terminator, resulting in the plasmid *pBJP5.* The final *pBJP6* plasmid was obtained by amplifying the *pyrG* marker of *Aspergillus nidulans* with two primers (FwpGAN-ISceIPmeI and RevpGAN-ISceIPmeI) from a plasmid and cloning into the *PmeI* site of *pBJP5* (between the two 600-bp overlapping GFP sequences in the *GFPn*-GFPc** construct). The primers were designed to incorporate the *I-Sce*I restriction sites and the *PmeI* sites downstream and upstream of the *pyrG* marker during the PCR amplification, resulting in a PCR amplified fragment carrying the *pyrG* marker flanked by two *I-SceI* sites. The resulting plasmid *pBJP6* contains the *5'UTR cbh2-Pcbh1-GFPn*-I-SceI-pyrG-I-SceI-GFPc*-Tcbh2* construct as shown in FIG. 6. The sequence of the pBJP6 plasmid was confirmed by restriction analysis and sequencing.

The primer sequences used in this example are presented below and also as Table 2.
GSP1 (Forward) 5'-TCTAGAGGCTGTGCATTTCCGTTCTC-3' (SEQ ID NO:7)
GSP2 (Reverse) 5'-TGGTTACGGCAACAAACCTG-3' (SEQ ID NO:8)
   PP1 (Forward)
5'-CAGGTTTGTTGCCGTAACCAATTTGCCTGCTTGACCGACTG-3' (SEQ ID NO:9)
   PP2 (Reverse)
5'-GGAACGATGGGTTTGCGTCCATATGGGGTAAGTCACTTACGGCAGC-3' (SEQ ID NO:10)
GSP3 (Forward) 5'-CCATATGGACGCAAACCCATCGTTCC-3' (SEQ ID NO:11)
GSP4 (Reverses) 5'-GGTACCGGTTCACCGCCTTATGTGAG-3'(SEQ ID NO:12)
   FwpGAN-ISceIPmel (Forward)
5'-GGTTTAAACCTAGGGATAACAGGGTAATTCGCCCTTGCTCTAGATAAC-3'(SEQ ID NO:13)
   RevpGAN-ISCELPmel (Reverse)
5'-GGTTTAAACCTAGGGATAACAGGGTAATAATTCGCCCTTGACTAGTGC-3'(SEQ ID NO:14)
GSP5 (Forward) 5'-GCGATCGCACGCAAACCCATCGTTCC-3' (SEQ ID NO:15)
GPS6 (Reverse) 5'- GCGATCGCGGTTCACCGCCTTATGTGAG -3' (SEQ ID NO:16)

**Table 2. Primers used in Example 2.**

| **Primer name** | **f/r** | **Sequence (5'to 3'oriented)** | **Template** |
|---|---|---|---|
| GSP1 | f | TCTAGA(XbaI)GGCTGTGCATTTCCGTTCTC | gDNA QM6a |
| GSP2 | r | TGGTTACGGCAACAAACCTG | gDNA QM6a |
| PP1 | f | *CAGGTTTGTTGCCGTAACCAA*TTTGCCTGCTTGACCGACTG | gDNA QM6a |
| PP2 | r | GGAACGATGGGTTTGCGTCCATATGG(NdeI) GGTAAGTCACTTACGGCAGC | gDNA QM6a |
| GSP3 | f | CCATATGG(NdeI)ACGCAAACCCATCGTTCC | gDNA QM6a |
| GSP4 | r | GGTACC(KpnI)GGTTCACCGCCTTATGTGAG | gDNA QM6a |
| FwpGAN - ISceIPmeI | f | GGTTTAAACC(PmeI)TAGGGATAACAGGGTAAT(IsceI) TCGCCCTTGCTCTAGATAAC | pCRpyrGAN |
| RevpGAN-ISceIPmeI | r | GGTTTAAACC(PmeI)TAGGGATAACAGGGTAAT(ISceI) AATTCGCCCTTGACTAGTGC | pCRpyrGAN |
| GSP5 | f | GCGATCGCACGCAAACCCATCGTTCC | gDNA QM6a |
| GSP6 | r | GCGATCGCGGTTCACCGCCTTATGTGAG | gDNA QM6a |

### 2. Design of a Marker Excision Method to Monitor I-SceI activity: Mechanism

To monitor the activity of the meganuclease *I-SceI* in *T. reesei,* the reporter construct *pBJP6* was used, and the method of the assay is depicted in FIG. 8.

As depicted schematically at the top of FIG. 7, *pBJP6* contains a *5'UTR cbh2-Pcbh1-GFPn*^{∗}*-I-SceI-pyrG-I-SceI-GFPc*^{∗}*-Tcbh2* construct. The inclusion of *5'UTR cbh2* and *Tcbh2* is used for targeted integration at the *cbh2* locus in a host cell. The two *I-SceI* recognition sites (the gray boxes with depicted scissors above) flanking the selection marker *pyrG* is used to monitor the activity of *I-SceI.*

The rationale of the strategy was that expression of active *I-SceI* in a *T. reesei* strain containing the *GFPn*^{∗}*-I-SceI-pyrG-I-SceI-GFPc*^{∗} construct would result in excision of the *pyrG* marker and repair of the double strand break by the GFP repeats (represented with the hatched boxes). A perfect repair via the repeats would result in a uridine auxotrophic strain (pyrG-) expressing GFP (depicted in FIG. 7).

### 3. Construction of T. reesei Strain Harboring the I-SceI Restriction Sites at the cbh2 Locus: Results

*pBJP6* was PCR amplified with the primers GSP1 and GSP4 (primer locations shown in FIG. 6), and the 7.5 kb PCR product/cassette for monitoring *I-SceI* activity as described above (the *5'UTR cbh2-Pcbh1-GFPn*^{∗}*-I-SceI-pyrG-I-SceI-GFPc*^{∗}*-Tcbh2* construct) is transformed to *T. reesei* strain P37ΔcbhIpyrG⁻²⁶ by PEG-mediated protoplast transformation method.

Uridine prototrophic transformants were purified and analysed by Southern blot for transformants in which the entire cassette was integrated at the *cbh2* locus. Based on initial diagnostic PCRs (results not shown), 9 transformants were selected for the Southern blot analysis. Three (3) different probes to select a single copy transformant and to confirm proper integration of the complete cassette into the genome at the *cbh2* locus.

The Southern blot analysis revealed 3 transformants harboring single copy of the complete *I-SceI* cassette at the *cbh2* locus (JP7.7, JP7.9 and JP7.12) (FIG. 8).

From the 6 remaining transformants 5 were shown to have multiples copies of the cassette integrated (JP7.10, JP7.11, JP7.13, JP7.14, JP7.15) or did not seem to have the complete cassette integrated into the genome (JP7.8).

Transformant JP7.7 was used in subsequent experiments.

### B. CONSTRUCTION OF I-SCEI EXPRESSING CONSTRUCT

### 1. Construction of I-SceI Expression Vector

A codon optimized synthetic gene of *I-SceI* sequence (GenBank Accession No. GU575293.1) was created for expression in *T. reesei* (Geneart, Germany). The synthetic *I-sceI* nucleotide sequence is shown below (SEQ ID NO:17):

ATGAAGAACATCAAGAAGAACCAGGTCATGAACCTCGGCCCCAACAGCAAGCTC
CTCAAGGAGTACAAGAGCCAGCTCATCGAGCTCAACATCGAGCAGTTCGAGGCC
GGCATCGGCCTCATCCTCGGCGACGCCTACATCCGCAGCCGCGACGAGGGCAAGA
CCTACTGCATGCAGTTCGAGTGGAAGAACAAGGCCTACATGGACCACGTCTGCCT
CCTCTACGACCAGTGGGTCCTCAGCCCCCCCACAAGAAGGAGCGCGTCAACCA
CCTCGGCAACCTCGTCATCACCTGGGGCGCCCAGACCTTCAAGCACCAGGCCTTC
AACAAGCTCGCCAACCTCTTCATCGTCAACAACAAGAAGACCATCCCCAACAACC
TCGTCGAGAACTACCTCACCCCCATGAGCCTCGCCTACTGGTTCATGGACGACGG
CGGCAAGTGGGACTACAACAAGAACAGCACCAACAAGAGCATCGTCCTCAACAC
CCAGAGCTTCACCTTCGAGGAGGTCGAGTACCTCGTCAAGGGCCTCCGCAACAA
GTTCCAGCTCAACTGCTACGTCAAGATCAACAAGAACAAGCCCATCATCTACATCG
ACAGCATGAGCTACCTCATCTTCTACAACCTCATCAAGCCCTACCTCATCCCCCAG
ATGATGTACAAGCTCCCCAACACCATCAGCAGCGAGACCTTCCTCAAGTAA

To construct a *I-SceI* expression vector, the *I-SceI* gene was cloned via a Gateway recombination according to the recommendations of the supplier (Life technologies, USA) into the telomeric plasmid *pTTT* as examplified in published patent application US20110020899. The lactose inducible promoter of *cbhI* gene was used to induce the expression of the *I-SceI* gene in the *pTTT* plasmid. In addition, the *pTTT* plasmid carried the *amdS* selection marker of *A. nidulans,* which allowed transformation and selection in *T. reesei* using acetamide as a nitrogen source

In the resultant expression cassette *pTTT-I-SceI* (FIG. 16) the lactose inducible promoter of the *cbhI* gene drives the expression of the *I-SceI* gene. In addition, the *pTTT* plasmid carried the *amdS* selection marker of *A. nidulans,* which allowed transformation and selection in *T. reesei* using acetamide as a nitrogen source.

### 2. I-SceI Expression in T. reesei Induces DSB at the Targeted Chromosomal Locus

The *pTTT-I-SceI* construct was used to transform strains containing a landing *I-SceI* site as well as a negative control strain which does not contain a landing *I-SceI* site. After transformation, *amdS* positive transformants were purified on Minimal medium, plated containing glucose and acetamide as nitrogen-source and point inoculated in the middle of a 9 cm Petri dish containing 2% glucose (non-inducing condition) or 2% lactose (inducing condition) as a carbon source. The transformation result is shown in FIG. 9.

As shown in FIG. 9, induction by lactose in the strains expressing the *I-SceI* and containing the *I-SceI* cassette results in colonies displaying sector formation. The sectors are indicated with arrows point to regions with no growth. This sectoring was interpreted as *I-SceI* activity that generated a double strand break which was repaired via the GFP repeats leading to a loss of the *pyrG* marker.

As a consequence of losing the *pyrG* marker, this part of the colony would no longer grow on plates without uridine giving rise to the sectors. As shown in FIG. 9, these sectors were mostly present when lactose was used as a carbon source, although sectors were also observed on the glucose plates, indicating that, even on glucose, some *I-SceI* was expressed.

Sectoring on lactose was observed for transformants expressing the *pTTT-ISceI* but not for the control strains (FIG. 9). When uridine was added to the lactose plates, no sectoring was visible anymore indicating that the sectors were caused by losing the *pyrG* marker (FIG. 9). The sectoring of the transformants on lactose plates demonstrated that the S. *cerevisiae I-SceI* was expressed and active in *T. reesei.*

Recombination was also monitored in liquid cultures. Transformants were grown in liquid culture in Minimal medium with uridine and *I-SceI* expression was induced by Sophorose. Microscopy observation of the resulting transformants is shown in FIG. 10. Fluorescent microscopy of hyphae showed GFP fluorescent indicating reconstitution of a functional GFP as a consequence of a loopout. As GFP was observed only in strains harboring the I-SceI expression cassette (JP7.7.12, JP7.7.14, and JP7.7.16) upon induction, the result further indicates that *I-SceI* was actively expressed upon induction (FIG. 10).

### C. IMPROVED TRANSFORMATION AND TARGETED INTEGRATION EFFICIENCY OF A GLUCOAMYLASE

### 1. Construction of Glucoamylase Expression Cassette For Targeted Integration

The plasmid *ptrex6gGA*/*wt* as described in U.S. Patent No. US8138321, which carries the wild-type glucoamylase gene of *T. reesei* under control of the *cbhI* promoter was used as a starting point to construct the glucoamylase expression cassette which can integrate at the *I-SceI* landing site.

To allow homologous integration of the glucoamylase cassette at the *I-SceI* landing site, the *cbh2* terminator region (*Tcbh2*) was cloned in *ptrex6gGA*/*wt* (FIG. 11). The *Tcbh2* was obtained by PCR using the primers GSP5 and GSP6 (as provided in Table 2) and the genomic DNA of QM6a as template.

The PCR product was digested with *AsiSI* and cloned into the same restriction sites of *ptrex6gGA*/*wt,* to give plasmid *pJP8.* The 10-kb glucoamylase expression cassette was cut out from *pJP8* with *PsiI* and used for transformation. Transformants were selected via the *alS* marker that confers resistance to chlorimuron ethyl.

### 2. Transformation Frequency. Stability, And Efficiency of Targeted Integration Under I-SceI Expression

To determine whether a DSB (double-stranded break) mediated by *I-SceI* can increase transformation and recombination efficiency in *T. reesei,* the glucoamylase expression cassette was transformed for targeted integration as a linear fragment into strain carrying the *I-SceI* restriction sites and *I-SceI* expression cassette (JP7.7.12).

Transformants were plated out of selective medium (containing chlorimuron ethylsubstrate) and containing glucose (*I-SceI* non-inducing condition) or containing a mix of lactose and glucose (*I-SceI* inducing condition). As a negative control, a strain carrying only *I-SceI* restriction sites but not *I-SceI* expression cassette (JP7.7) was also transformed with the same amount of the linear plasmid. Next, the stability of transformants obtained in each group of transformation was analyzed. The transformation results and stability analysis results are shown in Table 3 and FIG. 12.

**Table 3. The effect of I-SceI induction on number and stability of transformants**

| Strain | c-source | # of primary transformants^{a} | % stable transformants^{b} | % Gla positive transformants^{c} | % Gla positive pyrG⁻ transformants^{d} | Homologous recombination efficiency^{e} |
|---|---|---|---|---|---|---|
| JP7.7 (Control) | Lactose | 32 | 17/32 (53 %) | Not tested | Not tested | Not tested |
| JP7.7 (Control) | Glucose | 36 | 21/36 (58 %) | 12/40 (30 %) | 2/12(16%) | 2/12(16 %) |
| JP7.7.12 (pTTT-ISceI) | Lactose | > 226 | 45/50 (90 %) | 22/40 (55 %) | 15/22 (68 %) | 15/22(68%) |
| JP7.7.12 (pTTT-ISceI) | Glucose | 70 | 22/50 (44 %) | 15/40 (37 %) | 7/15(46%) | 7/15(46%) |

| | | | | | | |
|---|---|---|---|---|---|---|
| ^{a} Number of primary transformants on transformation plate (TrMMsorb + uridine + chlorimuron ethyl) containing 1 % of lactose and 1 % glucose referred to as induced and non-induced expression of *I-SceI* respectively. Control transformations have been carried out using the transformation medium using the parental strain JP7.7. ^{b} Primary transformants were purified on TrMM + uridine + chlorimuron ethyl. The stability of the transformants is defined by their ability to growth in selective TrMM after double purification. Stable transformants grow well on these selective plates. Abortive transformants (not stable) do not grow. ^{c} Stable transformants were tested for glucoamylase activity in a microtiter based growth/activity assay. Transformants showing Gla activity above the background were considered as Gla positive transformants. ^{d} Gla positive transformants were tested for the pyrG phenotype by inoculating spores of each transformant on TrMM with uridine or TrMM without uridine. Strains were considered pyrG minus (uridine auxotroph) when growing on TrMM+uridine, but not growing om TrMM without uridine. ^{e} GlaA expressing and pyrG minus strain were analyzed by Southern blot and confirmed an integration pattern indicative of homologous integration of the glaA expression cassette at the *I-SceI* landing site. | | | | | | |

Results in Table 3and FIG. 12 showed that *I-SceI* induction increased the number of transformants more than three-fold and six-fold as compare to non-induced condition and to the control respectively (Table 3). In addition, almost all of the JP7.7.12 transformants after induction of *I-SceI* were stable (90%), while only 44% of JP7.7.12 transformants without the induction of *I-SceI* were stable, and only 53% or 58% of the negative control strain JP7.7's tranformants (with no I-SceI expression cassette) were stable(Table 3). This demonstrates that expression of *I-SceI* increased the transformation efficiency and also increased stability of transformants.

About 40 stable transformants from each group were screened for glucoamylase production by growing purified transformants on NREL medium in 24 well MTPs that slowly released lactose from a porous matrix, as descrbed in the above example. About 55% of the transformants were expressing glucoamylase in *I-SceI* induced condition, as versus 37 % and 30% in non-induced *I-SceI* condition and to the control respectively.

The transformants expressing glucoamylase were subsequently analyzed for their *pyrG* phenotype. Targeted integration of the glucoamylase expression cassette at the intended locus via a double cross over was expected to result in chlorimuron ethyl resistance (alS+) and a loss of *the pyrG* marker, resulting in uridine auxotrophy (FIG. 11).

As is shown in Table 3, when *I-SceI* is induced, 68% of transformants expressing glucoamylase were integrated in a targeted manner, versus only 46% and 16%, respectively, in non-induced *I-SceI* and in the control.

Southern blot analysis of the selected *pyrG* minus transformants expressing glucoamylase showed that they contained the GlaA expression cassette at the *I-SceI* landing site as expected (FIG. 13). Strains not expressing glucoamylase or expressing glucoamylase but not being *pyrG* minus showed altered integration patterns (FIG. 13).

### 3. Comparison of Glucoamylase Expression Between Targeted vs. non-Targeted Integration

To compare the variability of glucoamylase activity of transformants with a targeted integrated glucoamylase expression cassette to those derived from random integration, strains were grown in microtiter plates and the medium was tested for glucoamylase activity.

Each transformant was grown independently in three microtiter plate wells and the average glucoamylase activity of this biological triplicate was determined and gave high reproducible results with a standard deviation <10%.

As shown in FIG. 14, transformants with targeted integration of the glucoamylase cassette displayed less variability in glucoamylase expression (between 0.9 and 1.35 relative units of glucoamylase activity) compared to the expression of non-targeted integration of the glucoamylase cassette (FIG. 14). Clearly the non-targeted integration of the expression cassette gave rise to significant variation in GA activity levels among transformants.

In conclusion, this data demonstrated that the double stranded break created by *I-SceI* stimulated targeted integration of the cassette containing the gene of interest and increased the homogeneity of protein expression among transformants.

### D. IMPROVED TRANSFORMATION & TARGETED INTEGRATION OF A GLUCOAMYLASE EXPRESSION CASETTE AFTER CREATING A DSB BY DIRECT ADDITION OF I-SCEI TO THE PROTOPLAST MIXTURE

### 1. Targeted Integration via I-SceI Addition

Targeted integration mediated by *I-SceI* has been also tested by adding directly the *I-SceI* restriction enzyme into the mixture of protoplast carrying the *I-SceI* restriction sites (JP7.7) with linearized glucoamylase targeted integrated cassette (*pJP8*).

Briefly, to 200 µL of JP7.7 protoplasts different amounts of *I-SceI* enzyme plus linearized *pJP8* cassette was added and incubated for 15 minutes at room temperature followed by 20 minutes incubation on ice. *I-SceI* was obtained from Thermo scientific. The transformation was done by PEG mediated protoplast method by adding to the protoplast, *I-SceI* and DNA mixture, IX final concentration of the enzyme buffer (e.g. with 270 µL total volume of protoplast, I-SceI and DNA mixture, 30 µL of the 10 X enzyme buffer is added). After the 35 min. of incubation, the protoplast mixture was plated on transformation plate containing 10 µM uridine and 5 µg/ml of Chlorimuron ethyl The result is summarized in Table 4:

**Table 4. Effect of I-SceI addition on efficiency of transformation and targeted integration.**

| I-SceI addition | # of primary transformants^{a} | % stable transformants^{b} | % Gla positive transformants ^{c} | % Gla positive pyrG⁻ transformants ^{d} | Homologous recombination efficiency^{e} |
|---|---|---|---|---|---|
| No *I-SceI* added | 19 | 13 (68%) | 3/13 (23%) | 0/3 (0%) | 0 |
| 25 U | 27 | n.d | n.d | n.d. | n.d |
| 50U | 56 | 18/20 (90 %) | 14/18 (77 %) | 11/14 (78 %) | 78 % |
| 100U | 70 | 20/20 (100 %) | 14/20 (70 %) | 12/14 (85 %) | 85 % |
| 100U+FOA ^{f} | 31 | 22/30 (73 %) | 22/22 (100 %) | 22/22 (100 %) | 100 % |

| | | | | | |
|---|---|---|---|---|---|
| ^{a} Number of Primary transformants on transformation plate (trMMsorb +uridine +chlorimuron ethyl). Numbers of primary transformants are shown for the same protoplastbatch to be able to compare efficiencies. Results for atypical experiment are shown. ^{b} Primary transformants were purified on trMM+ uridine + chlorimuron ethyl. Stable transformants grow well on these plates. Abortive transformants (not stable) do not grow. ^{c} Stable transformants were tested for glucoamylase activity in a microtiter based growth/activity assay. Transformants showingGlaactivity above the background were considered as Gla positive transformants. ^{d} Gla positive transformants were tested for the pyrGphenotype by inoculating spores of each transformant on trMM with uridine or trMM without uridine. Strains were considered pyrG minus (uridine auxotroph) when growing on trMM+uridine, but not growing om trMM without uridine. ^{e} GlaA expressing and pyrG minus strain were analysed to Southern blot and confirmed an integration pattern indicative of homologous integration of the glaA expression cassette at the *I*-*SceI* landing site. ^{f} Transformants were directly plated on trMM- Sorb containing uridine, chlorimuron ethyl medium and 0.8 mg/ml 5'fluoro-orotic acid (5'FOA). Colonies were purified once on trMM containing uridine and Als substrate medium. | | | | | |

As shown in Table 4, direct addition of *I-SceI* enzyme increased transformation frequency and the number of targeted integrated transformants. The number of transformants obtained increased by 3-fold when 100 Units of *I-SceI* was added to the protoplasts.

A significant number of primary transformants were found to be stable when *I-SceI* was added.

Both the number of transformants and the percentage of stable transformants increased with increasing *I-SceI* concentrations. About ∼75% of the transformants contained the Gla expression cassette in the presence of *I-SceI* while in the absence of *I-SceI,* only ∼25% of the transformants displayed glucoamylase activity.

In most of the transformants expressing glucoamylase the expression cassette was integrated via homologous recombination event (78 % and 85 % of transformants analyzed when adding 50 Units and 100 Units of *I-SceI* respectively). None of the transformants expressing glucoamylase from the control transformation (without adding *I-SceI*) were targeted integrated (Table 4).

By combining *I-SceI* addition with 0.8 mg/mL of FOA in the transformation plate, we were able to directly select the targeted integrated transformants (Table 4). After purification of the FOA resistant transformants all stable transformants tested expressed glucoamylase and the glucoamylase expression cassette was integrated properly at the *cbh2* locus via homologous recombination (FIG. 9).

### 2. Genetic Characterization and Comparison of Glucoamylase Expression, Comparing Targeted vs. Non-Targeted Integration Using I-SceI Expression

To compare the variability of glucoamylase activity of transformants with a targeted integrated glucoamylase expression cassette to those derived from random integration, strains were grown in microtiter plates and the medium was tested for glucoamylase activity.

As shown in FIG. 15, transformants with targeted integration of the glucoamylase cassette obtained from adding *I-SceI* directly displayed less variability in glucoamylase expression compared to the expression of random transformants. Transformants obtained by using the FOA selection also showed a similar expression pattern, indicating that the selection of FOA and the possible mutational effect of FOA did not affect the expression level of glucoamylase.

### EXAMPLE 3: Construction of Other Stable T. reesei Strains Using REMI

### A. TRANSFORMATION OF T. REESEI WITH TWO DIFFERENT DNA FRAGMENTS

Two different linear DNA fragments were constructed to express another protein of interest and introduced into *T. reesei* by co-transformation. One fragment had the pyr2 gene as selectable marker, the other fragment had the amdS gene as selectable marker. Transformations were performed without any restriction enzyme or with the restriction enzyme SwaI or PacI. Transformants were selected simultaneously for the presence of both markers. Several transformations were performed to determine the effect of inclusion of varying amounts of different restriction enzymes during transformation compared with no added restriction enzyme. Below is a summary of the result showing the numbers of transformants obtained and the % that were stable.

Table 5. Results of *T. reesei* co-transformation with two different linear DNA fragments.

| Restriction enzyme | # transformants positive for both selectable markers | # stable transformants (% of stable transformants) |
|---|---|---|
| None | 30 | 1 (3.3 %) |
| 0.25 uL (2.5 units) SwaI | 31 | 3 (9.7%) |
| 1 uL (10 units) SwaI | 49 | 6 (12.2%) |
| 0.5 uL (5 units) PacI | 77 | 8 (10.4%) |
| 2 uL (20 units) PacI | 44 | 7 (15.9%) |

### B. TRANSFORMATION OF T. REESEI TREATED WITH MULPIPLE RESTRICTION ENZYMES

To see the effect of using multiple restriction enzymes on transformation efficiency and stability of transformants, a *T. reesei* strain was transformed with a PCR fragment comprising a lytic cellulose monooxygenase (EG4)-encoding sequence and a pyr2 selectable marker not treated with any restriction enzyme, treated with 20 units of only AsiSI, treated with 7 units each of AsiSI, PacI and SwaI, or treated with 7 units each of AsiSI, PacI and Pmel. The transformation was performed according to a standard PEG protocol as described hereinabove, and transformants were grown on Minimal medium plates with 1M sorbitol. The results are shown in FIGS. 17A and 17B. With digestion by the same total amount of restriction enzymes, digesting with three different enzymes increased the percentage of stable *T. reesei* transformants (to 80%) as compared to digesting with only one restriction enzyme (50%), although it appeared to lower the transformation efficiency.

### REFERENCES

1. Akashi H, Matsumoto S, Taira K. Gene discovery by ribozyme and siRNA libraries. Nat Rev Mol Cell Biol 2005; 6:413-22.
2. Armaleo D, Ye GN, Klein TM, Shark KB, Sanford JC, Johnston SA. Biolistic nuclear transformation of Saccharomyces cerevisiae and other fungi. Curr Genet 1990; 17:97-103.
3. Aw R, Polizzi KM. Can too many copies spoil the broth? Microb Cell Fact. 2013; 12:128.
4. Campbell EI1, Unkles SE, Macro JA, van den Hondel C, Contreras R, Kinghorn JR. Improved transformation efficiency of Aspergillus niger using the homologous niaD gene for nitrate reductase. Curr. Genet. 1989; 16:53-56.
5. Cavaco-Paulo A and Gübitz GM. Textile Processing with Enzymes, 2003, 1st Edition
6. Chelikani P, Fita I, Loewen PC. Diversity of structures and properties among catalases. Cell Mol Life Sci. 2004; 61(2):192-208.
7. de Groot MJ, Bundock AP, Hooykaas PJJ, Beijersbergen AGM. Agrobacterium tumefaciens-mediated transformation of filamentous fungi. Nat Biotechnol 1998; 16:839-42.
8. Duyvesteijn RGE, Van Wijk R, Boer Y, RepM, Cornelissen BJC, HaringMA. Frp1 is a Fusarium oxysporum F-box protein required for pathogenicity on tomato. MolMicrobiol 2005; 57:1051-63.
9. Hinnen A, Hicks JB, Fink GR. Transformation of yeast. Proc Natl Acad Sci U S A 1978; 75:1929-33.
10. Imazaki I, Kurahashi M, Iida Y, Tsuge T. Fow2, a Zn(II)2Cys6-type transcription regulator, controls plant infection of the vascular wilt fungus Fusarium oxysporum. Mol. Microbiol. 2007; 63:737-53.
11. Ivanova C, Bååth JA, Seiboth B, Kubicek CP. Systems Analysis of Lactose Metabolism in Trichoderma reesei Identifies a Lactose Permease That Is Essential for Cellulase Induction. PLoS One. 2013; 8(5):
12. Jintao Xu, Guolei Zhao, Yanbo Kou, Weixin Zhang, Qingxin Zhou, Guanjun Chen and Weifeng Liu. Intracellular β-glucosidases CELla and CELlb are essential for cellulase induction on lactose in Trichoderma reesei. Eukaryotic Cell 2014; 13: 1001-1013.
13. Kanaar R, Hoeijmakers JJH, van Gent DC. Molecular mechanisms of DNA double strand break repair. Trends Cell Biol 1998; 8:483-9.
14. Karube I, Tamiya E, Matsuoka H. Transformation of Saccharomyces cerevisiae spheroplasts by high electric pulse. FEBS Lett 1985; 182:90-4.
15. Lu S, LyngholmL, Yang G, Bronson C, Yoder OC, Turgeon BG. Tagged mutations at the Tox1 locus of Cochliobolus heterostrophus by restriction enzyme-mediated integration. Proc Natl Acad Sci U S A 1994; 91:12649-53.
16. Madrid MP, Di Pietro A, Roncero MIG. Class V chitin synthase determines pathogenesis in the vascular wilt fungus Fusarium oxysporum and mediates resistance to plant defence compounds. Mol Microbiol 2003; 47:257-66.
17. MONTENECOURT B.S. CAN 1987, 1 - 20
18. Nalankilli.G.,Application of enzymes in eco-friendly wet processing of cotton, Colourage, 1998, XLV (10), 17-19.
19. Schiest RH, Petes TD. Integration of DNA fragments by illegitimate recombination in Saccharomyces cerevisiae. Proc Natl Acad Sci U S A 1991; 88:7585-9.
20. Seong KY, Hou ZM, TracyM, Kistler HC, Xu JR. Random insertional mutagenesis identifies genes associated with virulence in the wheat scab fungus Fusarium graminearum. Phytopathology 2005; 95:744-50.
21. SHEIR-NEISS ET AL. APPL. MICROBIOL. BIOTECHNOLOGY, 1984, 20:46 - 53
22. Shenai, V.A. and Saraf, N.M. Technology of Finishing, 1990,Vol. X.II Edition
23. Tanaka A, Shiotani H, Yamamoto M, Tsuge T. Insertional mutagenesis and cloning of the genes required for bio-synthesis of the host-specific AK-toxin in the Japanese pear pathotype of Alternaria alternata. Mol Plant Microbe Interact 1999; 12:691-702.
24. Van Dyck E, Stasiak AZ, Stasiak A, West SC. Binding of double-strand breaks in DNA by human Rad52 protein. Nature 1999; 398:728-31.
25. Weld RJ, Plummer KM, Carpenter MA, Ridgway HJ. Approaches to functional genomics in filamentous fungi. Cell Res 2006; 16:31-44.
26. B. Chevalier, R.J. Monnat, Jr., B.L. Stoddard. The LAGLIDADG Homing Endonuclease Family, in Nucleic Acids and Molecular Biology, Vol. 16, Marlene Belfort (Ed.) "Homing Endonucleases and Inteins", Springer-Verlag Berlin Heidelberg 2005, pages 33-47.
27. T. Gaj, C. A. Gersbach, and C. F. Barbas III. ZFN, TALEN and CRISPR/Cas-based methods for genome engineering. Trends Biotechnol. 2013; 31(7): 397-405.

## Claims

1. A method of improving the stability of *Trichoderma* transformants such as to construct a genetically stable, transformed *Trichoderma* strain, the method comprising:
a) transforming *Trichoderma* cells using a mixture of linearized or circular DNA comprising one or more genes of interest and a plurality of restriction enzymes, wherein the restriction enzymes are capable of generating a double strand break (DSB) in the chromosomal DNA of the *Trichoderma* cells; and
b) selecting a genetically stable, transformed *Trichoderma* strain produced in a),
wherein the percentage of stable transformants with treatment of the restriction enzymes is higher than percentage of stable transformants obtainable without treatment of the restriction enzymes,
wherein the restriction enzymes recognize a restriction site of at least 8 base pairs and are selected from: PacI, Swal, Pmel, AscI, AsiSI, Fsel, NotI, Srfl, and Sgfl.

2. The method of claim 1, wherein
(i) the percentage of stable transformants with treatment or induction of the restriction enzymes is at least 20%, 30%, 40%, 50%, 60%, 70%, 80%, or 90% higher than the percentage of stable transformants without treatment or induction of the restriction enzymes, and optionally the percentage of stable transformants with treatment or induction of the restriction enzymes is at least 1 fold, 2 folds, 3 folds, 4 folds, 5 folds, or 6 folds higher than the percentage of stable transformants without treatment or induction of the restriction enzymes; and\or
(ii) the percentage of stable transformants with treatment or induction of the restriction enzymes is at least 5%, 10%, 15%, 20%, 30%, 40%, 50%, 60%, 70%, or 80%; and\or
(iii)transformation efficiency with treatment or induction of the restriction enzymes is at least 5%, 10%, 20%, 30%, 40%, 50%, 60%, 70%, 80%, or 90% higher than transformation efficiency without treatment or induction of the restriction enzymes.

3. The method of any preceding claim, wherein the restriction enzymes are capable of generating compatible cohesive ends in the chromosomal DNA of transformed *Trichoderma* cells.

4. The method of any preceding claim, wherein the *Trichoderma* cells are transformed with linearized DNA.

5. The method of any one of claims 1-3 wherein the *Trichoderma* cells are transformed with circular DNA.

6. The method of any preceding claim, wherein the gene of interest encodes a hemicellulase, a peroxidase, a protease, a cellulase, a xylanase, a lipase, a phospholipase, an esterase, a cutinase, a pectinase, a keratinase, a reductase, an oxidase, a phenol oxidase, a lipoxygenase, a ligninase, a pullulanase, a tannase, a pentosanase, a mannanase, a beta-glucanase, an arabinosidase, a hyaluronidase, a chondroitinase, a laccase, an amylase, a glucoamylase, a variant thereof, a functional fragment thereof, or a mixture of two or more thereof.

7. The method of any one of claims 1 to 5, wherein the gene of interest encodes a peptide hormone, a growth factor, a clotting factor, a chemokine, a cytokine, a lymphokine, an antibody, a receptor, an adhesion molecule, a microbial antigen, a variant thereof, a functional fragment thereof, or a mixture of two or more thereof.

8. The method of any one of claims 1 to 5, wherein the linearized or circular DNA further comprises a selective marker.

9. The method of claim 8, wherein the selective marker is als1, amdS, hygR, pyr2, pyr4, sucA, a bleomycin resistance marker, a blasticidin resistance marker, a pyrithiamine resistance marker, a chlorimuron ethyl resistance marker, a neomycin resistance marker, an adenine pathway gene, a tryptophan pathway gene, or a thymidine kinase marker.

10. The method of any preceding claim, wherein the method does not comprise using a DNA or RNA sequence that is homologous to a *Trichoderma* genomic sequence.

11. The method of any one of claims 1-9, wherein the method comprises using a DNA or RNA sequence that is homologous to a *Trichoderma* genomic sequence.

12. The method of claim 11, wherein the method comprises using a DNA sequence that is homologous to a *Trichoderma* genomic sequence which recombines with the *Trichoderma* chromosomal DNA by homologous recombination at a site cut by the restriction enzymes.

13. The method of any preceding claim, wherein the *Trichoderma* strain is a *Trichoderma reesei* strain.

## Patentansprüche

1. Verfahren zum Verbessern der Stabilität von *Trichoderma-Transformanten,* um derart einen genetisch stabilen, transformierten *Trichoderma-Stamm* aufzubauen, wobei das Verfahren umfasst:
a) Transformieren von *Trichoderma-Zellen* unter Verwendung einer Mischung von linearisierter oder zirkulärer DNA, umfassend ein oder mehrere Gene, die von Interesse sind, und einer Mehrzahl von Restriktionsenzymen, wobei die Restriktionsenzyme zum Erzeugen eines Doppelstrangbruchs (DSB) in der chromosomalen DNA der *Trichoderma-Zellen* in der Lage ist; und
b) Auswählen eines genetisch stabilen, transformierten *Trichoderma-*Stammes, der in a) erzeugt wurde,
wobei der prozentuale Anteil von stabilen Transformanten mit Behandlung der Restriktionsenzyme höher ist als der prozentuale Anteil von stabilen Transformanten, die ohne Behandlung der Restriktionsenzyme erhalten werden können,
wobei die Restriktionsenzyme einen Restriktionsort von mindestens 8 Basenpaaren erkennen und ausgewählt sind aus: PacI, SwaI, PmeI, AscI, AsiSI, FseI, NotI, SrfI und SgfI.

2. Verfahren nach Anspruch 1, wobei
(i) der prozentuale Anteil von stabilen Transformanten mit Behandlung oder Induktion der Restriktionsenzyme mindestens um 20%, 30%, 40%, 50%, 60%, 70%, 80% oder 90% höher ist als der prozentuale Anteil von stabilen Transformanten ohne Behandlung oder Induktion der Restriktionsenzyme ist und wahlweise der prozentuale Anteil von stabilen Transformanten mit Behandlung oder Induktion der Restriktionsenzyme mindestens um das 1fache, 2fache, 3fache, 4fache, 5fache oder 6fache höher ist, als der prozentuale Anteil von stabilen Transformanten ohne Behandlung oder Induktion der Restriktionsenzyme ist; und/oder
(ii) der prozentuale Anteil von stabilen Transformanten mit Behandlung oder Induktion der Restriktionsenzyme mindestens 5%, 10%, 15%, 20%, 30%, 40%, 50%, 60%, 70% oder 80% beträgt; und/oder
(iii) Effizienz der Transformation mit Behandlung oder Induktion der Restriktionsenzyme mindestens 5%, 10%, 20%, 30%, 40%, 50%, 60%, 70%, 80% oder 90% höher als Effizienz der Transformation ohne Behandlung oder Induktion der Restriktionsenzyme ist.

3. Verfahren nach einem der vorhergehenden Ansprüche, wobei die Restriktionsenzyme zum Erzeugen kompatibler kohäsiver Enden in der chromosomalen DNA von transformierten *Trichoderma-Zellen* in der Lage sind.

4. Verfahren nach einem der vorhergehenden Ansprüche, wobei die *Trichoderma-*Zellen mit linearisierter DNA transformiert werden

5. Verfahren nach einem der Ansprüche 1 bis 3, wobei die *Trichoderma-Zellen* mit zirkulärer DNA transformiert werden.

6. Verfahren nach einem der vorhergehenden Ansprüche, wobei das Gen, das von Interesse ist, eine Hemicellulase, eine Peroxidase, eine Protease, eine Cellulase, eine Xylanase, eine Lipase, eine Phospholipase, eine Esterase, eine Cutinase, eine Pektinase, eine Keratinase, eine Reduktase, eine Oxidase, eine Phenoloxidase, eine Lipoxygenase, eine Ligninase, eine Pullulanase, eine Tannase, eine Pentosanase, eine Mannanase, eine Beta-Glucanase, eine Arabinosidase, eine Hyaluronidase, eine Chondroitinase, eine Laccase, eine Amylase, eine Glucoamylase, eine Variante davon, ein funktionales Fragment davon oder eine Mischung von zwei oder mehreren davon codiert.

7. Verfahren nach einem der Ansprüche 1 bis 5, wobei das Gen, das von Interesse ist, ein Peptidhormon, einen Wachstumsfaktor, einen Gerinnungsfaktor, ein Chemokin, ein Cytokin, ein Lymphokin, einen Antikörper, einen Rezeptor, ein Adhäsionsmolekül, ein mikrobielles Antigen, eine Variante davon, ein funktionales Fragment davon oder eine Mischung von zwei oder mehreren davon codiert.

8. Verfahren nach einem der Ansprüche 1 bis 5, wobei die linearisierte oder zirkuläre DNA ferner einen selektiven Marker umfasst.

9. Verfahren nach Anspruch 8, wobei der selektive Marker alsl, amdS, hygR, pyr2, pyr4, sucA, ein Bleomycin Resistenzmarker, ein Blasticidin Resistenzmarker, ein Pyrithiamin Resistenzmarker, ein Chlorimuron-Ethyl Resistenzmarker, ein Neomycin Resistenzmarker, ein Adenin Pathway-Gen, ein Tryptophan Pathway-Gen oder ein Thymidinkinase-Marker ist.

10. Verfahren nach einem der vorhergehenden Ansprüche, wobei das Verfahren keine Verwendung einer DNA- oder RNA-Sequenz umfasst, die zu einer genomischen *Trichoderma*-Sequenz homolog ist.

11. Verfahren nach einem der Ansprüche 1 bis 9, wobei das Verfahren die Verwendung einer DNA- oder RNA-Sequenz umfasst, die zu einer genomischen *Trichoderma-Sequenz* homolog ist.

12. Verfahren nach Anspruch 11, wobei das Verfahren die Verwendung einer DNA-Sequenz umfasst, die zu einer genomischen *Trichoderma-Sequenz* homolog ist, die mit der chromosomalen *Trichoderma-DNA* durch homologe Rekombination an einem Ort rekombiniert, der durch die Restriktionsenzyme getrennt wurde.

13. Verfahren nach einem der vorhergehenden Ansprüche, wobei der *Trichoderma-*Stamm ein Stamm von *Trichoderma reesei* ist.

## Revendications

1. Procédé pour l'amélioration de la stabilité de transformants de *Trichoderma* de manière à construire une souche de *Trichoderma* transformée, génétiquement stable, le procédé comprenant :
a) la transformation de cellules de *Trichoderma* en utilisant un mélange d'ADN linéarisé ou circulaire comprenant un ou plusieurs gènes d'intérêt et une pluralité d'enzymes de restriction, dans lequel les enzymes de restriction sont capables de générer une cassure double brin (DSB) dans l'ADN chromosomique des cellules de *Trichoderma ;* et
b) la sélection d'une souche de *Trichoderma* transformée, génétiquement stable produite dans a),
dans lequel le pourcentage de transformants stables avec le traitement des enzymes de restriction est supérieur au pourcentage de transformants stables pouvant être obtenus sans le traitement des enzymes de restriction,
dans lequel les enzymes de restriction reconnaissent un site de restriction d'au moins 8 paires de bases et sont choisies parmi : PacI, SwaI, PmeI, AscI, AsiSI, FseI, NotI, SrfI et SgfI.

2. Procédé selon la revendication 1, dans lequel :
(i) le pourcentage de transformants stables avec le traitement ou l'induction des enzymes de restriction est au moins 20%, 30%, 40%, 50%, 60%, 70%, 80% ou 90% supérieur au pourcentage de transformants stables sans le traitement ou l'induction des enzymes de restriction et, optionnellement, le pourcentage de transformants stables avec le traitement ou l'induction des enzymes de restriction est au moins 1 fois, 2 fois, 3 fois, 4 fois, 5 fois ou 6 fois supérieur au pourcentage de transformants stables sans le traitement ou l'induction des enzymes de restriction ; et/ou
(ii) le pourcentage de transformants stables avec le traitement ou l'induction des enzymes de restriction est d'au moins 5%, 10%, 15%, 20%, 30%, 40%, 50%, 60%, 70% ou 80% ; et/ou
(iii) le rendement de transformation avec le traitement ou l'induction des enzymes de restriction est au moins 5%, 10%, 20%, 30%, 40%, 50%, 60%, 70%, 80% ou 90% supérieur au rendement de transformation sans le traitement ou l'induction des enzymes de restriction.

3. Procédé selon l'une quelconque des revendications précédentes, dans lequel les enzymes de restriction sont capables de générer des extrémités cohésives compatibles dans l'ADN chromosomique de cellules de *Trichoderma* transformées.

4. Procédé selon l'une quelconque des revendications précédentes, dans lequel les cellules de *Trichoderma* sont transformées avec un ADN linéarisé.

5. Procédé selon l'une quelconque des revendications 1-3, dans lequel les cellules de *Trichoderma* sont transformées avec un ADN circulaire.

6. Procédé selon l'une quelconque des revendications précédentes, dans lequel le gène d'intérêt est codant pour une hémicellulase, une peroxydase, une protéase, une cellulase, une xylanase, une lipase, une phospholipase, une estérase, une cutinase, une pectinase, une kératinase, une réductase, une oxydase, une phénol oxydase, une lipoxygénase, une ligninase, une pullulanase, une tannase, une pentosanase, une mannanase, une bêta-glucanase, une arabinosidase, une hyaluronidase, une chondroïtinase, une laccase, une amylase, une glucoamylase, un variant de celles-ci, un fragment fonctionnel de celles-ci ou un mélange de deux ou plus de celles-ci.

7. Procédé selon l'une quelconque des revendications 1 à 5, dans lequel le gène d'intérêt est codant pour une hormone peptidique, un facteur de croissance, un facteur de coagulation, une chimiokine, une cytokine, une lymphokine, un anticorps, un récepteur, une molécule d'adhésion, un antigène microbien, un variant de ceux-ci, un fragment fonctionnel de ceux-ci ou un mélange de deux ou plus de ceux-ci

8. Procédé selon l'une quelconque des revendications 1 à 5, dans lequel l'ADN linéarisé ou circulaire comprend en outre un marqueur sélectif.

9. Procédé selon la revendication 8, dans lequel le marqueur sélectif est als1, amdS, hygR, pyr2, pyr4, sucA, un marqueur de résistance à la bléomycine, un marqueur de résistance à la blasticidine, un marqueur de résistance à la pyrithiamine, un marqueur de résistance au chlorimuron-éthyl, un marqueur de résistance à la néomycine, un gène de voie d'adénine, un gène de voie de tryptophane ou un marqueur de thymidine kinase.

10. Procédé selon l'une quelconque des revendications précédentes, où le procédé ne comprend pas l'utilisation d'une séquence d'ADN ou d'ARN qui est homologue à une séquence génomique de *Trichoderma.*

11. Procédé selon l'une quelconque des revendications 1 à 9, où le procédé comprend l'utilisation d'une séquence d'ADN ou d'ARN qui est homologue à une séquence génomique de *Trichoderma.*

12. Procédé selon la revendication 11, où le procédé comprend l'utilisation d'une séquence d'ADN qui est homologue à une séquence génomique de *Trichoderma* qui se recombine avec l'ADN chromosomique de *Trichoderma* par une recombinaison homologue à un site coupé par les enzymes de restriction.

13. Procédé selon l'une quelconque des revendications précédentes, dans lequel la souche de *Trichoderma* est une souche de *Trichoderma reesei.*
